# EUROPEAN PATENT SPECIFICATION

(11) **EP 3 338 630 B1**
(45) Date of publication and mention of the grant of the patent: **17.06.2020**
(21) Application number: 15902200.3
(22) Date of filing: 21.08.2015
(51) Int. Cl.: A61B 5/11, A61B 7/00

(54) **SWALLOWING MOVEMENT MONITORING SENSOR**
SCHLUCKBEWEGUNGSÜBERWACHUNGSSENSOR
CAPTEUR DE SURVEILLANCE DE MOUVEMENT DÉGLUTITION

(43) Date of publication of application: 27.06.2018
(73) Proprietor: Sumitomo Electric Industries, Ltd., Osaka-shi, Osaka 541-0041 (JP); Mitachi, Seiko, Kunitachi-shi, Tokyo 186-0003 (JP)
(72) Inventor: MITACHI, Seiko, Kunitachi-shi Tokyo 186-0003 (JP); KUWA, Kazuhiro, Osaka-shi Osaka 554-0024 (JP); HOSOYA, Toshifumi, Osaka-shi Osaka 554-0024 (JP); HAJI, Tomoyuki, Okayama-shi Okayama 701-1334 (JP)
(74) Representative: Boult Wade Tennant LLP
(86) International application number: PCT/JP2015/073530
(87) International publication number: WO 2017/033228

(56) References cited:
- EP-A1- 1 787 582
- WO-A1-2007/026858
- JP-A- H08 584
- JP-A- 2011 004 968
- JP-A- 2012 200 300
- YUKI UNDO ET AL.: 'Application of F-SAS sensor to dysphagia measurement' EIC PROCEEDINGS OF THE 2015 IEICE GENERAL CONFERENCE 24 February 2015, page 136, XP009504786
- TOMOYUKI HAJI ET AL.: 'Swallowing Sounds Recorded through the Ear : Preliminary Study of Normal Subjects' THE JOURNAL OF THE JAPANESE BRONCHO-ESOPHAGOLOGICAL SOCIETY vol. 66, no. 1, 10 February 2015, pages 13 - 19, XP009504776

## Description

### TECHNICAL FIELD

This invention relates to a swallowing movement monitoring sensor which measures swallowing movement of a subject.

### BACKGROUND ART

In the aging society in recent years, elderly people live a longer life whereas they suffer from various physical influences by deterioration in functions of the whole body involved with aging. Dysphagia represents one of such influences. Dysphagia refers to impairment of a swallowing function which is an action to swallow food and/or drinks.

Endoscopic swallowing examination, videofluoroscopic examination of swallowing, and swallowing pressure examination have conventionally widely been used as methods of assessing a swallowing function. A repetitive saliva swallowing test, a modified water swallowing test, and a food test have been conducted as screening of the dysphagia.

Recently, cervical auscultation of swallowing sound and assessment with a saturation monitor have also been attempted (see NPDs 1 and 2).

YUKI UNDO ET AL.: "Application of F-SAS sensor to dysphagia measurement", EIC PROCEEDINGS OF THE 2015 IEICE GENERAL CONFERENCE, 24 January 2015, page 136 describes a sleep apnea sensor that uses an optical fibre to capture changes in lateral pressure due to breathing as signal light changes using microbending loss.

JP H08584 A provides a vital signal detection and vital signal detector.

JP 2012 200300 A relates to a swallowing movement measurement system.

JP 2011 004968 A provides a deglutition activity monitoring device, deglutition activity monitoring system, biological information recording device and deglutition activity monitoring program.

WO 2007/026858 A1 relates to an organic activity monitoring method, optical ire type flat body sensor and garment type optical fiber flat body sensor used in this and human body attaching type optical fiber type flat body sensor.

EP 1 787 582 A1 describes a device and method for measuring continuous swallowing motion.

### CITATION LIST

### NON PATENT DOCUMENT

NPD 1: Ryuzaburo Higo et al., "Various Methods to Assess Swallowing Function," 2002, the Japan Journal of Logopedics and Phoniatrics Vol. 43, pp. 460-466
NPD 2: Tomoyuki Haji et al., "Swallowing Sounds Recorded through the Ear: Preliminary Study of Normal Subjects," 2015, the journal of the Japan Broncho-Esophagological Society, Vol. 66 (1), pp. 13-19

### SUMMARY OF INVENTION

### TECHNICAL PROBLEM

The endoscopic swallowing examination, the videofluoroscopic examination of swallowing, and the swallowing pressure examination above allow objective assessment of the swallowing function, whereas they require expensive equipment and staff and rely on an invasive test.

In the repetitive saliva swallowing test, the modified water swallowing test, and the food test, swallowing is sensed normally based on visual recognition and palpation of laryngeal elevation. Therefore, though those tests are more simplified than the examination above, they are disadvantageously low in accuracy and objectivity, and in particular, they are low in sensitivity to minor dysphagia.

Cervical auscultation of swallowing sound is a non-invasive test method, however, it may be difficult to distinguish swallowing sound from mastication sound, sound in the mouth, and sound of laryngeal elevation irrelevant to swallowing. It is difficult to detect minor aspiration. Therefore, this method remains as auxiliary diagnosis. Though assessment with the saturation monitor is also non-invasive and simple, relation between variation in saturation and aspiration is yet to be studied and this assessment method thus lacks reliability.

An object of one manner of the present invention is to provide a swallowing movement monitoring sensor which can non-invasively and objectively measure swallowing movement with high sensitivity with a simplified configuration.

### SOLUTION TO PROBLEM

A swallowing movement monitoring sensor according to the present invention is defined in claim 1 and is a sensor which measures swallowing movement of a subject. The swallowing movement monitoring sensor includes a first outside shape variation sensor attached to a laryngeal portion of the subject. The first outside shape variation sensor is configured to detect variation in outside shape of the laryngeal portion associated with swallowing movement.

### ADVANTAGEOUS EFFECTS OF INVENTION

According to the above, a swallowing movement monitoring sensor which can non-invasively and objectively measure swallowing movement with high sensitivity with a simplified configuration can be provided.

### BRIEF DESCRIPTION OF DRAWINGS

Fig. 1 is a schematic configuration diagram of a swallowing movement monitoring sensor according to an embodiment.
Fig. 2 is a diagram showing attachment of an optical fiber sheet and a microphone shown in Fig. 1 to a subject.
Fig. 3 is a diagram showing exemplary measurement of swallowing sound with the microphone.
Fig. 4 is a schematic configuration diagram of an optical fiber sheet representing one embodied example of an outside shape variation sensor shown in Fig. 1.
Fig. 5 is a plan view showing one exemplary placement of an optical fiber on the optical fiber sheet.
Fig. 6 is a diagram showing exemplary measurement of swallowing movement with the optical fiber sheet.
Fig. 7 is a schematic configuration diagram showing one example of a swallowing movement monitoring sensor system configured with the swallowing movement monitoring sensor according to the present embodiment.
Fig. 8 is a diagram showing a result of measurement when a subject swallows saliva consecutively three times.
Fig. 9 is a diagram showing a result of measurement when a subject swallows a small amount of water.
Fig. 10 is a diagram showing a result of measurement when a subject swallows jelly.
Fig. 11 is a diagram showing a result of measurement when a subject swallows saliva consecutively three times.
Fig. 12 is a diagram showing an output waveform shown in Fig. 11 as being partially enlarged.
Fig. 13 is a diagram showing a result of measurement when POF is employed.
Fig. 14 is a diagram showing a result of measurement when GI-SiO₂ is employed.
Fig. 15 is a diagram showing a result of measurement when HSFF is employed.
Fig. 16 is a plan view showing exemplary placement of an optical fiber on an optical fiber sheet.
Fig. 17 is a plan view showing exemplary placement of an optical fiber on an optical fiber sheet.
Fig. 18 is a plan view showing exemplary placement of an optical fiber on an optical fiber sheet.
Fig. 19 is a plan view showing exemplary placement of an optical fiber on an optical fiber sheet.
Fig. 20 is a diagram illustrating a method of attaching an optical fiber sheet.
Fig. 21 is a diagram showing a result of measurement with the attachment method shown in Fig. 21.
Fig. 22 is a diagram illustrating a method of attaching an optical fiber sheet.
Fig. 23 is diagram showing a result of measurement with the attachment method shown in Fig. 22.
Fig. 24 is a diagram showing a detection signal from an optical fiber sheet measured with a digital oscilloscope.
Fig. 25 is a diagram showing an output waveform from an optical fiber sheet subjected to simple moving average processing.
Fig. 26 is a schematic configuration diagram of a swallowing movement monitoring sensor system according to a fourth modification.
Fig. 27 is a diagram showing a result of measurement with the swallowing movement monitoring sensor system shown in Fig. 26.
Fig. 28 is a schematic configuration diagram of a swallowing movement monitoring sensor system according to a fifth modification.
Fig. 29 is a schematic configuration diagram of a swallowing movement monitoring sensor system according to the fifth modification.
Fig. 30 is a diagram showing a configuration of a swallowing movement monitoring sensor 1A according to a sixth modification.
Fig. 31 is a diagram showing attachment of outside shape variation sensors and the microphone shown in Fig. 30 to a subject.
Fig. 32 is a schematic configuration diagram showing one example of a swallowing movement monitoring sensor system configured with the swallowing movement monitoring sensor according to the sixth modification.

### DESCRIPTION OF EMBODIMENTS

### [Description of Embodiment of the Present Invention]

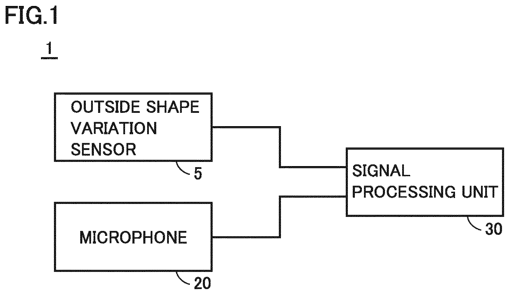

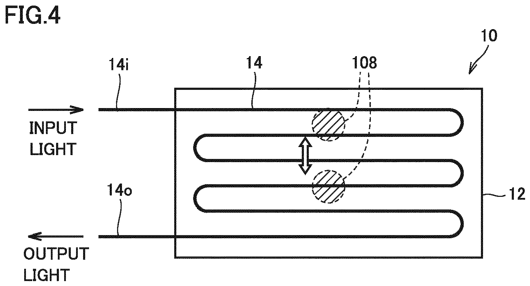

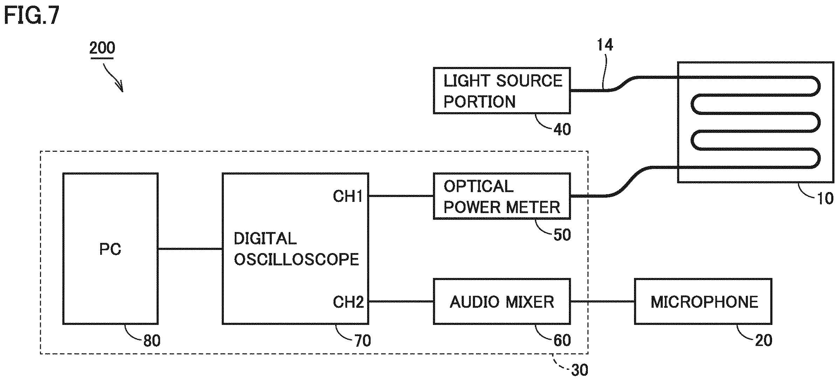

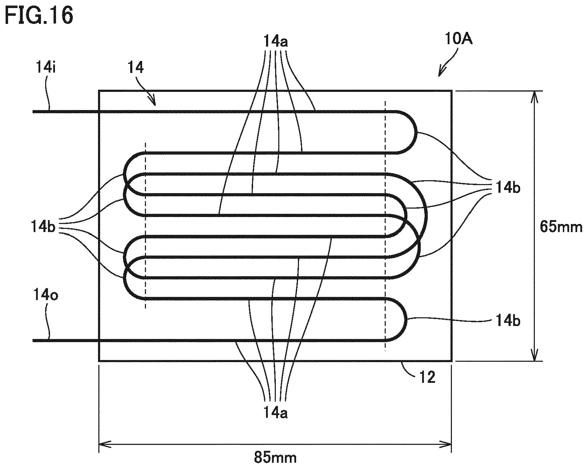

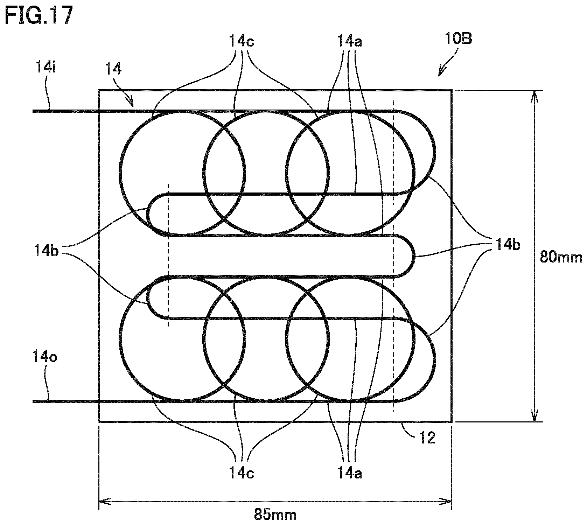

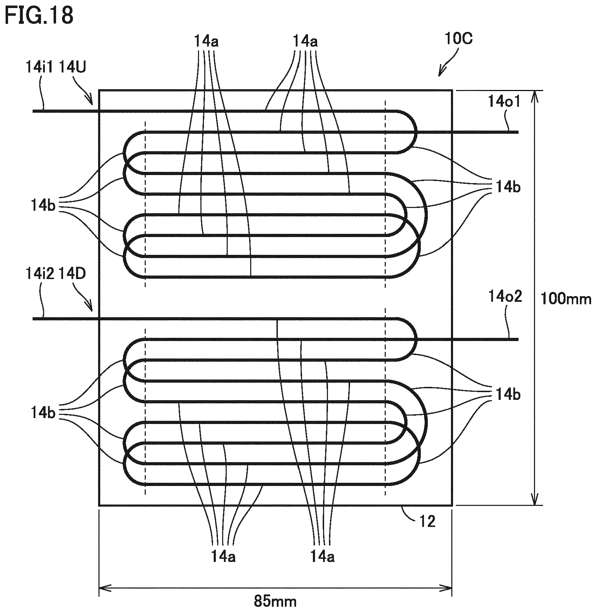

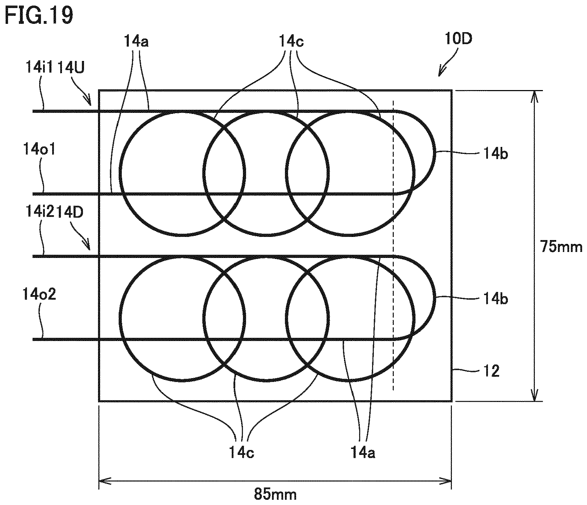

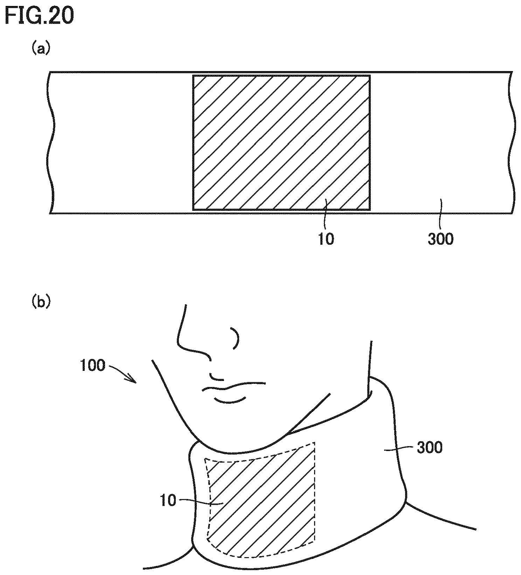

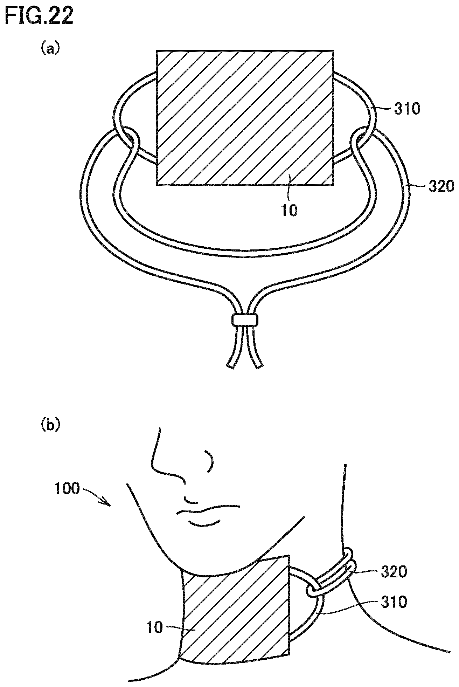

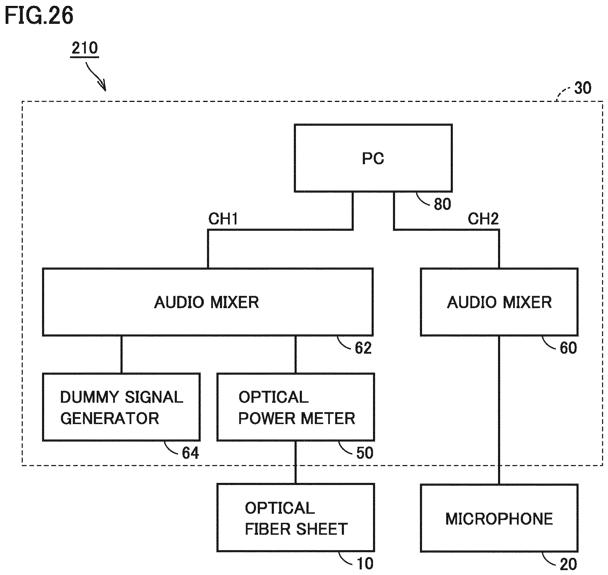

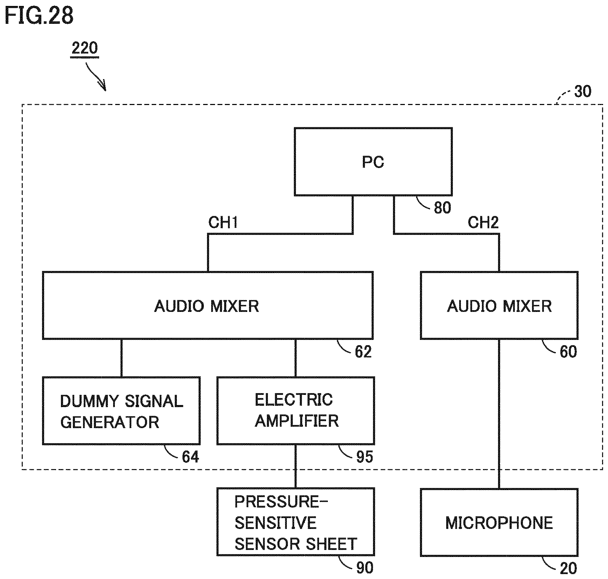

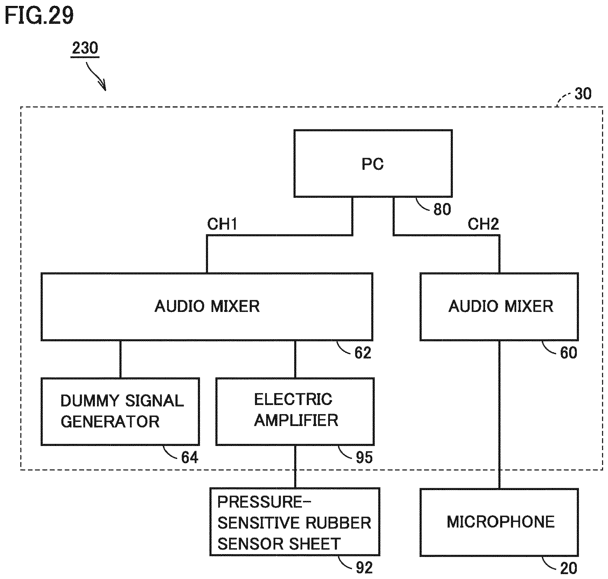

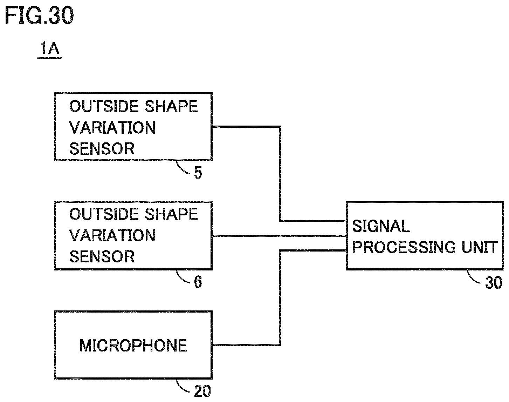

Embodiments of the present invention will initially be listed and described.
(1) A swallowing movement monitoring sensor 1 (see Fig. 1) according to the present invention is a sensor which measures swallowing movement of a subject. Swallowing movement monitoring sensor 1 includes a first outside shape variation sensor (an outside shape variation sensor 5) attached to a laryngeal portion of the subject. The first outside shape variation sensor (outside shape variation sensor 5) is configured to detect variation in outside shape of the laryngeal portion associated with swallowing movement. By doing so, swallowing movement can non-invasively and objectively be measured with a simplified configuration.
   The first outside shape variation sensor (outside shape variation sensor 5) includes an optical fiber sheet 10 (see Fig. 4) having an optical fiber 14 and a sheet-like member 12 supporting the optical fiber. Optical fiber sheet 10 is attached to the laryngeal portion. Optical fiber sheet 10 is configured to detect variation in quantity of light of a transmission signal beam based on a loss produced by a lateral pressure applied to optical fiber 14 from the laryngeal portion in association with variation in outside shape of the laryngeal portion involved with swallowing movement. By doing so, variation in outside shape of the laryngeal portion involved with swallowing movement can be measured in a non-invasive and simplified manner.
   According to the invention, optical fiber 14 (see Fig. 16) has a meandering portion formed from one optical fiber portion, the meandering portion being deformed to form a plurality of linear portions 14a extending in a first direction and a plurality of curved portions 14b connecting end portions of two linear portions 14a to each other. The optical fiber portion is deformed such that a third linear portion 14a located relatively downstream passes between first and second linear portions 14a located relatively upstream in the meandering portion.
   By doing so, a length of an optical fiber (an optical path length) can be increased and an interval between adjacent linear portions 14a can be shorter. Consequently, variation in transmission loss in response to movement of the laryngeal portion of a subject is great and hence sensitivity as the swallowing movement monitoring sensor can be improved.
(2) In the swallowing movement monitoring sensor according to (1), preferably, an interval between adjacent linear portions 14a is adjusted to at least two linear portions 14a in response to movement of the laryngeal portion.
(3) In the swallowing movement monitoring sensor according to (1), preferably, the optical fiber portion is deformed such that a plurality of annular portions 14c (see Fig. 17) aligned in the first direction are further formed in at least one 14a of the plurality of linear portions 14a in the meandering portion.
   By doing so, a contact point where optical fibers are in contact with each other is formed in a portion where annular portions 14c intersect with each other and a portion where annular portion 14c and linear portion 14a intersect with each other. Since a lateral pressure is produced in optical fiber 14 with this contact point serving as a point of load, a higher lateral pressure resulting from movement of the laryngeal portion is applied to optical fiber 14. Consequently, greater variation in quantity of light is caused in response also to slight movement of the laryngeal portion and hence sensitivity as the swallowing movement monitoring sensor can be improved.
(4) In the swallowing movement monitoring sensor according to (1) or (3), preferably, an optical fiber sheet 10C (see Fig. 18) or an optical fiber sheet 10D (see Fig. 19) further includes a plurality of input ends to which a plurality of transmission signal beams are input in parallel and a plurality of output ends which output the plurality of transmission signal beams in parallel. The optical fiber portion is placed between the input end and the output end which form a pair.
   By doing so, in each of the plurality of optical fibers 14 placed on common sheet-like member 12, a transmission loss is varied with application of a lateral pressure in accordance with movement of the laryngeal portion. There is a time lag in variation in transmission loss among the plurality of optical fibers 14. By detecting a time lag of difference in transmission loss, a speed of elevation movement (swallowing movement) of the laryngeal portion of a subject can be measured.
(5) In swallowing movement monitoring sensor 1 according to any of (1), (3) or (4), preferably, optical fiber 14 is a plastic optical fiber, a quartz-based optical fiber, or a plastic clad quartz glass core optical fiber. By doing so, a loss can be produced in transmission light by a lateral pressure applied to the optical fiber from the laryngeal portion. The optical fiber is more preferably a graded index type quartz-based optical fiber.
(6) In the swallowing movement monitoring sensor according to (5), preferably, optical fiber 14 is of a step index type, a graded index type, or a single mode type.
(7) In the swallowing movement monitoring sensor according to any of (1) or (3) to (6), preferably, optical fiber sheets 10 and 10A to 10D have a width in the first direction not smaller than 30 mm and not greater than 500 mm and a width in a second direction perpendicular to the first direction not smaller than 30 mm and not greater than 150 mm. The first direction is preferably a direction perpendicular to movement of the laryngeal portion and the second direction is preferably a direction in parallel to movement of the laryngeal portion. Then, regardless of the sex and an individual difference in length of a cervical portion of a subject, variation in outside shape of the laryngeal portion involved with swallowing movement can be measured with high sensitivity.
(8) In swallowing movement monitoring sensor 1 according to (7), preferably, optical fiber sheet 10 (see Fig. 20) is attached around a neck, with the laryngeal portion being defined as a center, with a fixing band 300. By doing so, optical fiber sheet 10 can be in intimate contact with the laryngeal portion and hence variation in outside shape of the laryngeal portion can be measured with high sensitivity.
(9) In the swallowing movement monitoring sensor according to (7), preferably, optical fiber sheet 10 (see Fig. 22) is attached around a neck, with the laryngeal portion being defined as a center, with an elastic member in a form of a string (elastic strings 310 and 320) attached to opposing lateral side surfaces of the optical fiber sheet. By doing so, optical fiber sheet 10 can be in intimate contact with the laryngeal portion and hence variation in outside shape of the laryngeal portion can be measured with high sensitivity.
(10) In the swallowing movement monitoring sensor according to (7), preferably, optical fiber sheet 10 is attached around a neck, with the laryngeal portion being defined as a center, with an annular net made of a stretchable material. By doing so, optical fiber sheet 10 can be in intimate contact with the laryngeal portion and hence variation in outside shape of the laryngeal portion can be measured with high sensitivity.
   In the swallowing movement monitoring sensor according to (1), first outside shape variation sensor 5 (see Fig. 28) may include a pressure-sensitive sensor sheet 90 having a capacitance-type pressure-sensitive element and a sheet-like member supporting the pressure-sensitive element and attached to the laryngeal portion. By doing so, variation in outside shape of the laryngeal portion involved with swallowing movement can be measured in a non-invasive and simplified manner.
   In the swallowing movement monitoring sensor according to (1), first outside shape variation sensor 5 (see Fig. 29) may include a pressure-sensitive rubber sensor sheet 92 having pressure-sensitive rubber and a sheet-like member supporting the pressure-sensitive rubber and attached to the laryngeal portion. By doing so, variation in outside shape of the laryngeal portion involved with swallowing movement can be measured in a non-invasive and simplified manner.
(11) Swallowing movement monitoring sensor 1 according to any of (1) or (3) to (10) preferably further includes a microphone 20 attached in an ear of the subject. Microphone 20 is configured to detect swallowing sound produced in the ear in association with swallowing movement. By doing so, variation in outside shape of the laryngeal portion involved with swallowing movement and swallowing sound can simultaneously be captured with a simplified configuration. Therefore, swallowing movement can non-invasively and objectively be measured with high sensitivity.
(12) In the swallowing movement monitoring sensor according to (11), preferably, microphone 20 is implemented by a lavaliere condenser microphone having a diameter not smaller than 1.5 mm and not greater than 5.0 mm. By doing so, swallowing sound can be picked up with high sensitivity.
(13) In the swallowing movement monitoring sensor according to (11) or (12), preferably, swallowing movement monitoring sensor 1 further includes a signal processing unit 30 configured to process a detection signal output from each of the first outside shape variation sensor (outside shape variation sensor 5) and microphone 20.
   By doing so, a swallowing function can objectively be assessed by analyzing results of measurement of variation in outside shape of the laryngeal portion involved with swallowing movement and swallowing sound. Therefore, by measuring swallowing movement of a normal example and a dysphagia example with the swallowing movement monitoring sensor system, an objective indicator in dysphagia screening can be presented. Consequently, convenience in clinical use can be enhanced.
(14) In swallowing movement monitoring sensor 1 according to (13), preferably, signal processing unit 30 (see Fig. 7) includes a display (a digital oscilloscope 70 and a PC 80) configured to show an output waveform of the detection signal from microphone 20 and an output waveform of the detection signal from the first outside shape variation sensor (outside shape variation sensor 5 as being aligned on an identical time axis. By doing so, by comparing two output waveforms, swallowing movement can accurately be detected and analyzed.
(15) In swallowing movement monitoring sensor 1 according to (14), preferably, the display (digital oscilloscope 70 and PC 80) shows the output waveform obtained by subjecting the detection signal from the first outside shape variation sensor (outside shape variation sensor 5) to simple moving average processing. By doing so, since noise superimposed on the detection signal can be removed, swallowing movement can be measured with high sensitivity.
(16) In swallowing movement monitoring sensor 1 according to (14) or (15) preferably, signal processing unit 30 (see Fig. 26) includes an audio input portion configured to be capable of digital sampling of the detection signal from microphone 20. The audio input portion receives input of the detection signal from the first outside shape variation sensor on which a dummy signal having a frequency handleable by the audio input portion is superimposed, together with the detection signal from the microphone. Output waveforms of the detection signal from the microphone and the detection signal from the first outside shape variation sensor are subjected to digital sampling in the audio input portion and thereafter shown on the display as being aligned on the identical time axis.
   When a digital oscilloscope is not used, signal processing unit 30 inputs the detection signal from the first outside shape variation sensor (outside shape variation sensor 5) on which a dummy signal representing an alternating-current signal is superimposed by an audio mixer 62 to a first audio input of an audio input portion of the display (PC 80). The dummy signal has a frequency handleable by an audio amplifier. Signal processing unit 30 further inputs the detection signal from microphone 20 to a second audio input of the audio input portion of the display (PC 80). Thus, output waveforms obtained by subjecting these two signals to digital sampling are shown on the display (PC 80) as being aligned on the identical time axis. By doing so, two output waveforms can be compared with each other by combining simplified apparatuses and hence swallowing movement can readily be detected and analyzed. When the display (PC 80) includes no audio input portion, the same function can be achieved by an external audio input unit.
(17) A swallowing movement monitoring sensor 1A (see Fig. 30) according to (1) preferably further includes a second outside shape variation sensor 6 attached to a chest portion or an abdominal portion of the subject. Second outside shape variation sensor 6 is configured to detect variation in outside shape of a chest associated with swallowing movement.
   By doing so, in addition to variation in outside shape of the laryngeal portion involved with swallowing movement and swallowing sound of a subject, variation in outside shape of the chest involved with swallowing movement can simultaneously and non-invasively be captured with a simplified configuration. Thus, swallowing movement and a respiration pattern in swallowing can non-invasively and objectively be measured. Consequently, the swallowing function can objectively be assessed.
(18) In swallowing movement monitoring sensor 1A (see Fig. 30) according to (17), preferably, second outside shape variation sensor 6 includes an optical fiber sheet having an optical fiber and a sheet-like member supporting the optical fiber. The optical fiber sheet is attached to the chest portion or the abdominal portion. The optical fiber sheet is configured to detect variation in quantity of light of a transmission signal beam based on a loss produced by a lateral pressure applied to the optical fiber from the chest portion or the abdominal portion in association with variation in outside shape of the chest involved with swallowing movement. By doing so, variation in outside shape of the chest involved with swallowing movement can be measured in a non-invasive and simplified manner. A pressure-sensitive sensor sheet or a pressure-sensitive rubber sensor sheet can be employed instead of an optical fiber sheet for second outside shape variation sensor 6, as in first outside shape variation sensor 5.

### [Details of Embodiment of Present Invention]

An embodiment of the present invention will be described hereinafter with reference to the drawings. In the drawings below, the same or corresponding elements have the same reference characters allotted and description thereof will not be repeated.

### <Configuration of Swallowing Movement Monitoring Sensor>

Fig. 1 is a schematic configuration diagram of a swallowing movement monitoring sensor according to an embodiment.

Referring to Fig. 1, swallowing movement monitoring sensor 1 according to the embodiment includes outside shape variation sensor 5, microphone 20, and signal processing unit 30.

Outside shape variation sensor 5 is attached to a laryngeal portion of a subject. Outside shape variation sensor 5 is configured to detect variation in outside shape of the laryngeal portion associated with swallowing movement. Details of outside shape variation sensor 5 will be described later.

Microphone 20 is a small-sized microphone which has a diameter approximately from 1.5 to 5.0 mm. For example, a lavaliere condenser microphone can be employed for microphone 20.

Signal processing unit 30 processes a detection signal output from each of outside shape variation sensor 5 and microphone 20. Details of signal processing unit 30 will be described later.

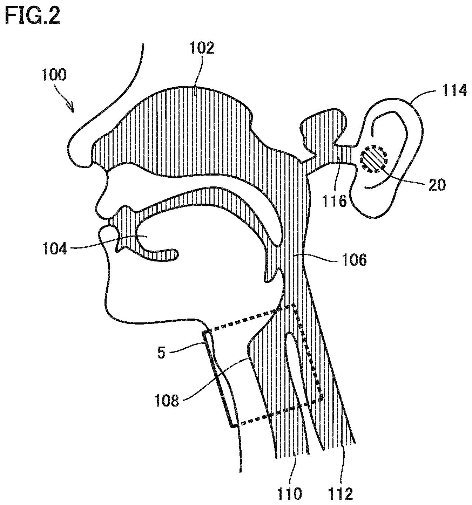

Fig. 2 is a diagram showing attachment of outside shape variation sensor 5 and microphone 20 shown in Fig. 1 to a subject 100. Fig. 2 schematically shows a skull portion of subject 100. A pharyngeal portion 106 is a pathway for air and food from nasal cavity 102 to esophagus 112 and trachea 110. A laryngeal portion 108 refers to a part of an airway including vocal cords around the Adam's apple.

As shown in Fig. 2, outside shape variation sensor 5 is attached around the neck of subject 100, with laryngeal portion 108 being defined as the center. For example, an optical fiber sheet can be employed for outside shape variation sensor 5. The optical fiber sheet includes an optical fiber and a sheet-like member which supports the optical fiber, and is configured to detect variation in outside shape of laryngeal portion 108 associated with swallowing movement. The optical fiber sheet corresponds to one embodied example of the "outside shape variation sensor."

Microphone 20 is attached to the inside of an ear 114 (in an external auditory meatus 116) of subject 100. Microphone 20 is configured to detect swallowing sound produced in the ear in association with swallowing movement.

A detection signal indicative of variation in outside shape of laryngeal portion 108 detected by outside shape variation sensor 5 and a detection signal indicative of swallowing sound detected by microphone 20 are both input to signal processing unit 30 (Fig. 1).

The present inventors have found that swallowing sound characterized by bimodal sounds of clicking sound containing a high-frequency component and a sound group containing a large amount of subsequent low-frequency components is produced in association with instantaneous opening of an auditory tube at the time of swallowing. The present inventors have further succeeded in recording swallowing sound from the inside of the ear (see, for example, NPD 2).

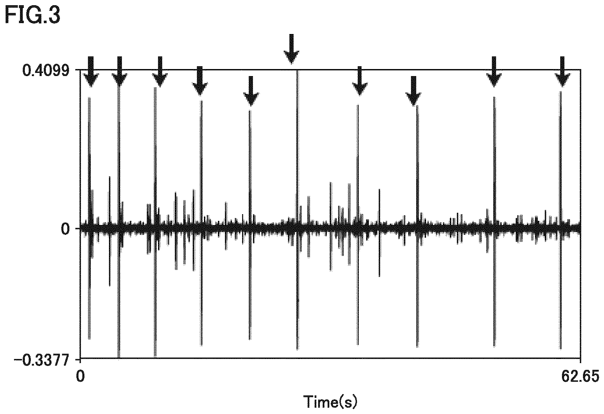

Fig. 3 is a diagram showing exemplary measurement of swallowing sound with microphone 20.

Fig. 3 shows an output waveform of a detection signal from microphone 20. The ordinate in Fig. 3 represents a voltage output from a lavaliere condenser microphone as a detection signal from microphone 20. The abscissa in Fig. 3 represents time.

The output waveform in Fig. 3 represents results of measurement when one subject (an adult male) swallows saliva consecutively ten times. Specifically, a lavaliere condenser microphone (a trade name of Countryman B06 manufactured by Countryman Associates, Inc.) having a diameter of 2.5 mm was inserted in an ear of the subject and a sound volume of a signal output from the condenser microphone was adjusted by an audio mixer (a trade name of Mackie 402-VLZ3 manufactured by Mackie).

As shown in Fig. 3, ten sharp audio waveforms (clicking sounds) in total were observed in the output waveform from microphone 20 (see arrows in the figure). Therefore, it can be seen that the number of times of swallowing and the number of clicking sounds match with each other.

With swallowing movement monitoring sensor 1 according to the present embodiment, swallowing sound is recorded from the inside of an ear and simultaneously variation in outside shape of the laryngeal portion associated with swallowing movement is detected. Swallowing movement can thus accurately be measured with a non-invasive and objective measurement method.

### (Configuration of Optical Fiber Sheet)

Fig. 4 is a schematic configuration diagram of optical fiber sheet 10 representing one embodied example of outside shape variation sensor 5 shown in Fig. 1.

Referring to Fig. 4, optical fiber sheet 10 includes optical fiber 14 and sheet-like member 12. Sheet-like member 12 is rectangular in a plan view. Optical fiber 14 is placed on sheet-like member 12.

Optical fiber 14 is partially fixed to sheet-like member 12, for example, with a double-faced tape. A material for sheet-like member 12 is not particularly limited so long as it can support optical fiber 14. Optical fiber 14 is not only placed on sheet-like member 12 but also can be embedded in sheet-like member 12.

Sheet-like member 12 has a length in a direction of width (a left-right direction in the figure) not smaller than 30 mm and not greater than 500 mm and a length in a direction of length (an up-down direction in the figure) not smaller than 30 mm and not greater than 150 mm. Then, regardless of the sex or an individual difference in length of the cervical portion of subjects, variation in outside shape of the laryngeal portion involved with swallowing movement can be measured with high sensitivity.

Optical fiber 14 is an optical transmission medium which includes a core, an outer layer on an outer side of the core which is called a clad, and a cover layer which covers the outer layer. A plastic optical fiber, a quartz-based optical fiber, or a plastic clad quartz glass core optical fiber can be employed for optical fiber 14. The plastic optical fiber is an optical fiber in which acrylic is employed for a core material and a fluorine resin is employed for a cladding material. The quartz-based optical fiber is an optical fiber in which quartz glass is employed for a core material and a cladding material. The plastic clad quartz glass core optical fiber is an optical fiber in which quartz glass is employed for a core material and a resin is employed for a cladding material.

A constant quantity of light continuously supplied from a not-shown light source portion is incident on an input end 14i of optical fiber 14. Light incident on input end 14i of optical fiber 14 is transmitted through optical fiber 14 and thereafter emitted from an output end 14o of optical fiber 14. A not-shown optical power meter (light reception portion) is connected to output end 14o of optical fiber 14. The optical power meter measures a quantity of light emitted from optical fiber 14.

As shown in Fig. 2, while optical fiber sheet 10 is attached to laryngeal portion 108 of subject 100, a lateral pressure is applied to optical fiber 14 in accordance with movement (variation in outside shape) of laryngeal portion 108. When subject 100 performs swallowing movement, laryngeal portion 108 exhibits movement (elevation and descent of laryngeal portion 108) as shown with an arrow in Fig. 4. Therefore, a lateral pressure applied to optical fiber 14 is varied with movement of laryngeal portion 108 involved with swallowing movement of subject 100.

In optical fiber 14, since a microbending loss is caused due to stress imposed by the lateral pressure, an excessive loss is caused in a transmission loss of optical fiber 14. Optical fiber sheet 10 according to the present embodiment makes use of this characteristic, and swallowing movement of subject 100 can be measured by measuring variation in transmission loss due to variation in lateral pressure applied to optical fiber 14 resulting from swallowing movement.

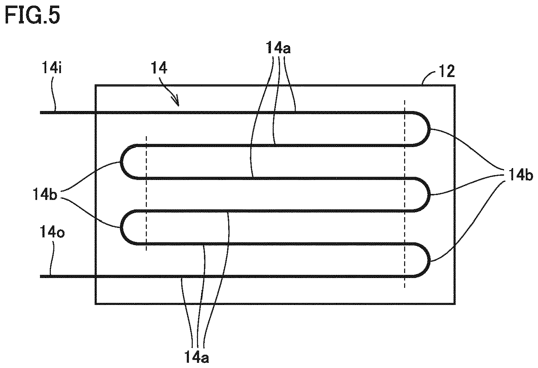

Fig. 5 is a plan view showing one exemplary placement of optical fiber 14 on optical fiber sheet 10.

Referring to Fig. 5, optical fiber 14 is placed on sheet-like member 12 as if it were drawn with a single stroke. Optical fiber 14 may be placed in any manner so long as it is placed as if it were drawn with a single stroke.

In the example in Fig. 5, a meandering portion formed by deforming one optical fiber portion is placed on sheet-like member 12. The meandering portion has a plurality of linear portions 14a which extend in a direction of width (a left-right direction in the figure) of sheet-like member 12. The direction of width of sheet-like member 12 corresponds to the "first direction." The first direction is set, for example, to a direction perpendicular to movement of the laryngeal portion (the direction shown with the arrow in Fig. 4).

The plurality of linear portions 14a are arranged as being aligned at an interval in the direction of length (the up-down direction in the figure) of sheet-like member 12. The direction of length of sheet-like member 12 corresponds to the "second direction." The second direction is set, for example, to a direction in parallel to movement of the laryngeal portion (the direction shown with the arrow in Fig. 4). An interval between adjacent linear portions 14a is adjusted such that a lateral pressure is applied to at least two linear portions 14a in response to movement of the laryngeal portion. The meandering portion further has a plurality of curved portions 14b which connect end portions of adjacent linear portions 14a to each other.

As set forth above, optical fiber sheet 10 according to the present embodiment makes use of variation in transmission loss caused by a lateral pressure and hence a longer optical path is more advantageous for improvement in sensitivity. According to the exemplary placement shown in Fig. 5, an optical path length is increased and hence sensitivity as a swallowing movement monitoring sensor can be improved.

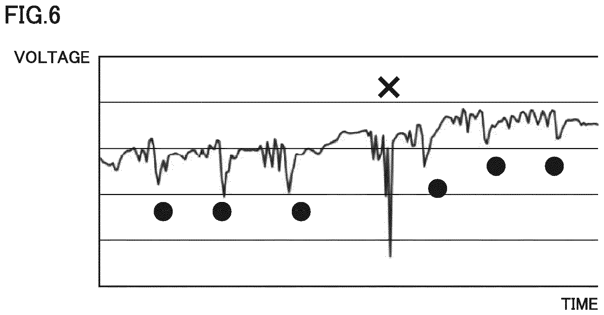

Fig. 6 is a diagram showing exemplary measurement of swallowing movement with optical fiber sheet 10. Fig. 6 shows an output waveform of a detection signal from optical fiber sheet 10. The output waveform represents a result of measurement when one subject (adult male) consecutively swallows saliva.

Specifically, optical fiber sheet 10 was constructed by placing a plastic optical fiber in accordance with the exemplary placement in Fig. 5 on rectangular sheet-like member 12 having a lateral width of 100 mm and a vertical width of 500 mm. Optical fiber sheet 10 was attached around the neck of a subject, with the laryngeal portion being defined as the center, with an annular net (a medical netted bandage) made of a stretchable material. In this state, a constant quantity of light was continuously incident from a light source portion on the input end of the plastic optical fiber and a quantity of light emitted from the output end of the plastic optical fiber was measured with the optical power meter.

The ordinate in Fig. 6 represents a quantity of received light (a voltage) measured with the optical power meter as a detection signal from optical fiber sheet 10 and the abscissa represents time.

Fig. 6 shows with a circular mark, timing of swallowing of saliva by the subject. It can be seen that each time the subject swallows saliva, a quantity of emitted light decreases. Fig. 6 further shows with a cross mark, timing of experimental touching to optical fiber sheet 10 with a finger. When optical fiber sheet 10 was touched with the finger, steep variation in waveform (lowering in quantity of emitted light) different from variation at the time of swallowing was observed. It was thus confirmed that optical fiber sheet 10 responded with good sensitivity to movement of the laryngeal portion of the subject.

### (Swallowing Movement Monitoring Sensor System)

A method of measuring swallowing movement with a swallowing movement monitoring sensor system including swallowing movement monitoring sensor 1 according to the present embodiment and a result of measurement will be described below.

### <Configuration of Swallowing Movement Monitoring Sensor System>

Fig. 7 is a schematic configuration diagram showing one example of the swallowing movement monitoring sensor system configured with swallowing movement monitoring sensor 1 according to the present embodiment.

Referring to Fig. 7, a swallowing movement monitoring sensor system 200 includes optical fiber sheet 10 (outside shape variation sensor), microphone 20, a light source portion 40, an optical power meter 50, an audio mixer 60, digital oscilloscope 70, and personal computer (PC) 80. Optical power meter 50, audio mixer 60, digital oscilloscope 70, and PC 80 implement one embodied example of "signal processing unit 30" shown in Fig. 1.

As described with reference to Fig. 2, in measuring swallowing movement, optical fiber sheet 10 representing outside shape variation sensor 5 is attached to the laryngeal portion of a subject and microphone 20 is attached to the inside of an ear of the subject.

Light source portion 40 continuously outputs a constant quantity of light. A light emitting diode (LED) is suitably employed for light source portion 40 because it can supply emission light in a stable manner, it is small in size and light in weight, and it is low in amount of heat generation and power consumption.

Optical power meter 50 receives emission light transmitted from optical fiber 14 and measures a quantity of received light. The measured quantity of received light (an amount of attenuation) is varied with a lateral pressure applied to optical fiber sheet 10 in accordance with movement of the laryngeal portion of the subject. Therefore, it can be determined that movement of the laryngeal portion is great when variation in quantity of received light is great and movement of the laryngeal portion is small when variation in quantity of received light is low. Optical power meter 50 converts the measured quantity of received light into an electric signal and inputs the resultant electric signal to a channel CH1 of digital oscilloscope 70.

A detection signal indicating swallowing sound detected by microphone 20 is input to audio mixer 60. Audio mixer 60 adjusts a volume of the detected swallowing sound and inputs the adjusted detection signal to a channel CH2 of digital oscilloscope 70.

Digital oscilloscope 70 receives an output signal from optical power meter 50 at channel CH1 and receives an output signal from audio mixer 60 at channel CH2. Digital oscilloscope 70 holds variation over time (output waveform) of the output signal from optical power meter 50 and the output signal from audio mixer 60, for example, as voltage waveform data. Digital oscilloscope 70 transmits two output waveforms to PC 80.

Digital oscilloscope 70 can also show two output waveforms as being aligned on the same time axis. Digital oscilloscope 70 corresponds to one embodied example of the "display" configured to be able to show an output waveform of a detection signal from microphone 20 and an output waveform of a detection signal from optical fiber sheet 10 as being aligned on the same time axis.

PC 80 analyzes two pieces of output waveform data transmitted from digital oscilloscope 70 by driving analysis software installed in advance. A result of analysis by PC 80 is shown on a display apparatus (not shown) such as a CRT or a printer. PC 80 corresponds to one embodied example of the "analysis portion."

### <Method of Measurement of Swallowing Movement and Result of Measurement>

Swallowing movement of a subject was measured with swallowing movement monitoring sensor system 200 shown in Fig. 7. One example of the result of measurement will be described below.

### (First Result of Measurement)

In measurement, a 22-year old adult male without dysphagia was adopted as a subject and optical fiber sheet 10 was attached to the laryngeal portion of the subject. Optical fiber sheet 10 was constructed such that a plastic optical fiber was placed in accordance with the exemplary placement in Fig. 5 on rectangular sheet-like member 12 having a lateral width of 80 mm and a vertical width of 100 mm. Optical fiber sheet 10 was attached around the neck of the subject, with the laryngeal portion being defined as the center, with a tracheal cannula holder for fixing a tracheal cannula.

Microphone 20 was attached to the inside of an ear of the subject. A lavaliere condenser microphone (a trade name of Countryman B06 manufactured by Countryman Associates, Inc.) having a diameter of 2.5 mm was employed as microphone 20. The condenser microphone was passed through an ear plug and inserted in the ear of each subject to thereby record swallowing sound. The condenser microphone was connected to audio mixer 60 (a trade name of Mackie 402-VLZ3 manufactured by Mackie).

Drinking water (for example, water) and soft food (for example, jelly) were selected as food and beverage to be swallowed.

In swallowing movement monitoring sensor system 200, red LED light (having a wavelength of 650 nm) from light source portion 40 was incident on the input end of optical fiber 14 and a quantity of light emitted from optical fiber 14 was measured with optical power meter 50.

A detection signal from optical fiber sheet 10 and a detection signal from microphone 20 were input to channels CH1 and CH2 of digital oscilloscope 70 (a trade name of UDS-5202 manufactured by JDS Inc.), respectively. PC 80 determined correspondence between output waveforms of the two detection signals by starting up analysis software which can measure the two detection signals on the same time axis.

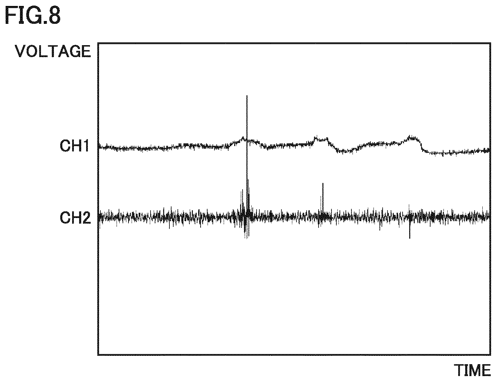

Figs. 8 to 10 show results of measurement. In each figure, the ordinate represents a quantity of received light (voltage) measured with optical power meter 50 and an output voltage from the lavaliere condenser microphone, and the abscissa represents time. The quantity of received light (voltage) measured by optical power meter 50 corresponds to the detection signal from optical fiber sheet 10. The output voltage from the lavaliere condenser microphone corresponds to the detection signal from microphone 20.

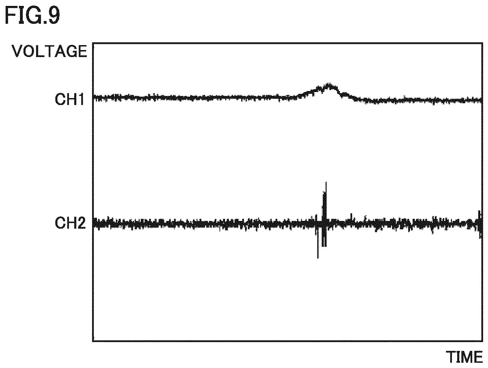

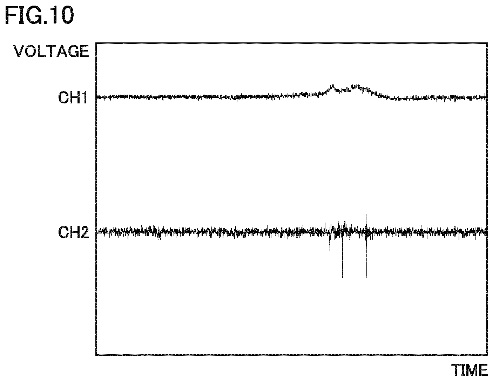

Fig. 8 shows a result of measurement when the subject swallowed saliva consecutively three times. Fig. 9 shows a result of measurement when the subject swallowed a small amount of water. Fig. 10 shows a result of measurement when the subject swallowed jelly. In each figure, an upper portion shows the output waveform at channel CH1 (the detection signal from optical fiber sheet 10) and a lower portion shows the output waveform at channel CH2 (the detection signal from microphone 20).

In each of Figs. 8 to 10, variation occurred in each of the output waveform from optical fiber sheet 10 and the output waveform from microphone 20. Timing of occurrence of variation in output waveform from optical fiber sheet 10 well matches with timing of occurrence of variation in output waveform from microphone 20. Therefore, it was confirmed that optical fiber sheet 10 and microphone 20 each captured swallowing movement of the subject.

Based on comparison between the output waveforms from optical fiber sheet 10 shown in Figs. 9 and 10, it was confirmed that a manner of variation in output waveform was different between swallowing of a liquid (water) and a solid (jelly). Specifically, it was confirmed that sharp variation in waveform could be captured when a liquid was swallowed than when a solid was swallowed.

### (Second Result of Measurement)

In swallowing movement monitoring sensor system 200, digital oscilloscope 70 was changed to a digital oscilloscope (a trade name of OWON VDS3104 manufactured by Lilliput) different from the digital oscilloscope (a trade name of UDS-5202 manufactured by JDS Inc.) used in the first result of measurement. The detection signal from optical fiber sheet 10 and the detection signal from microphone 20 were input to channels CH1 and CH2 of the new digital oscilloscope, respectively.

In measurement, four adult males without dysphagia were adopted as subjects and optical fiber sheet 10 was attached to the laryngeal portion of each subject.

A graded index type quartz-based optical fiber (GI-SiO₂) was employed for optical fiber 14 of optical fiber sheet 10. GI-SiO₂ is a quartz-based optical fiber having such a graded index that an index of refraction has a distribution in symmetry with respect to a central axis from the center of the core toward the clad.

Drinking water (for example, tea) and soft food (for example, almond jelly) were selected as food and beverage to be swallowed.

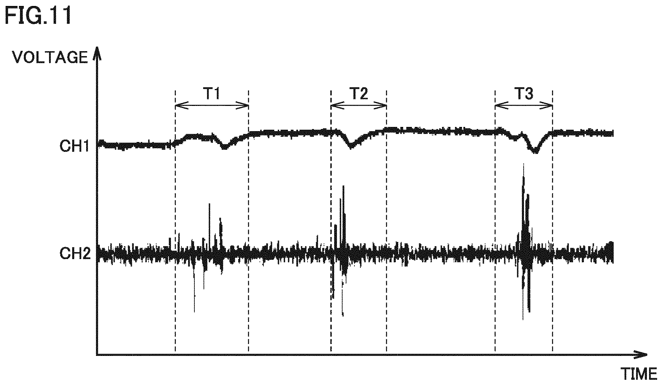

Fig. 11 shows a result of measurement when one subject swallowed saliva consecutively three times. The ordinate in Fig. 11 represents a quantity of received light (voltage) measured with optical power meter 50 and an output voltage from the lavaliere condenser microphone. The abscissa in Fig. 11 represents time.

Referring to Fig. 11, CH1 represents an output waveform of the detection signal from optical fiber sheet 10 and CH2 represents an output waveform of the detection signal from microphone 20.

Three sharp audio waveforms (clicking sounds) in total were observed in the output waveform from microphone 20 at times T1, T2, and T3, and it can be seen that the number of times of swallowing and the number of clicking sounds match with each other.

Decrease in quantity of received light was observed at each of times T1, T2, and T3 when the clicking sounds were observed in the output waveform from optical fiber sheet 10. It can thus be seen that a transmission loss of optical fiber 14 is varied each time the subject swallows saliva.

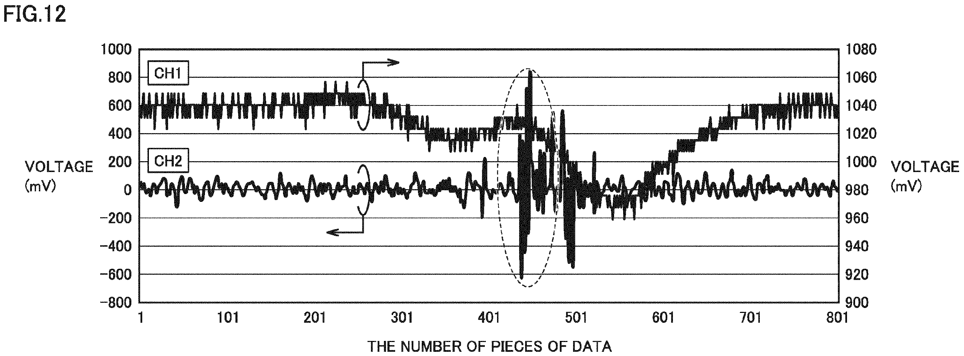

It was thus confirmed based on comparison between the output waveform from microphone 20 and the output waveform from optical fiber sheet 10 that timings of variation in waveform well match with each other. Fig. 12 shows in an enlarged manner, a waveform of a portion corresponding to third swallowing movement (corresponding to time T3 in Fig. 11), of the output waveforms shown in Fig. 11.

The waveform shown in Fig. 12 is extraction of sections of data numbers from 3500 to 4300 (the number of pieces of data being 800) from the output waveform data shown in Fig. 11 saved in a comma-separated values (CSV) format for graphing. It can be seen that, in a region surrounded by a dashed line in the figure, swallowing sound captured by microphone 20 and movement of the laryngeal portion captured by optical fiber sheet 10 correspond well to each other.

As described above, swallowing movement monitoring sensor 1 according to the present embodiment is configured such that optical fiber sheet 10 captures movement of the laryngeal portion of the subject whereas microphone 20 captures clicking sound (swallowing sound) emitted at the moment of opening of the auditory tube in the ear. It was confirmed from the results of measurement with swallowing movement monitoring sensor system 200 shown in Figs. 11 and 12 that optical fiber sheet 10 and microphone 20 captured swallowing movement of the subject in good agreement with each other.

### (Third Result of Measurement)

In measurement, sensitivity to swallowing of saliva, of three types of optical fiber sheets 10 different in optical fiber included therein was further compared, with four subjects in the second results of measurement being adopted.

In comparison, for swallowing movement monitoring sensor system 200 shown in Fig. 7, three types of optical fiber sheets 10 including any of a plastic optical fiber (POF), a graded index type quartz-based optical fiber (GI-SiO₂), and a step index type plastic clad quartz glass core optical fiber (high sensitive F-SAS fiber (HSFF)) were prepared. The step index type plastic clad quartz glass core optical fiber refers to a plastic clad quartz glass core optical fiber in which distribution of an index of refraction in the core is uniform.

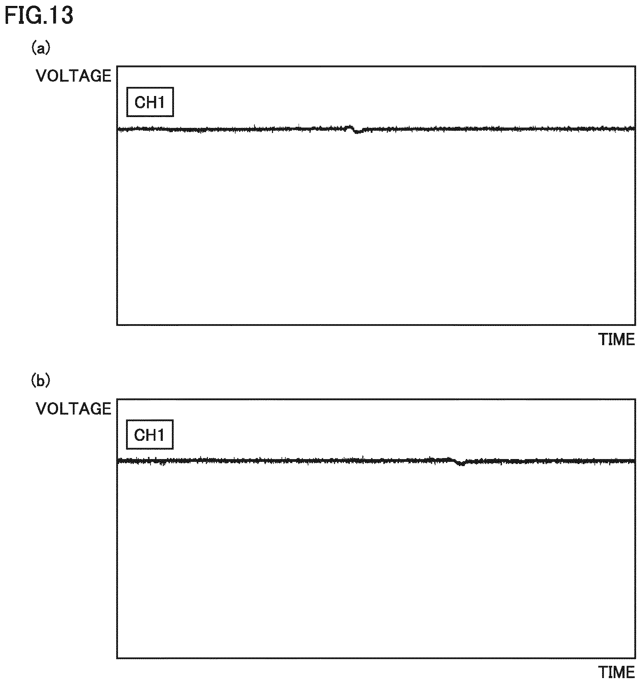

Swallowing movement was measured for each subject with three types of optical fiber sheets 10 and microphone 20. Figs. 13 to 15 show examples of results of measurement.

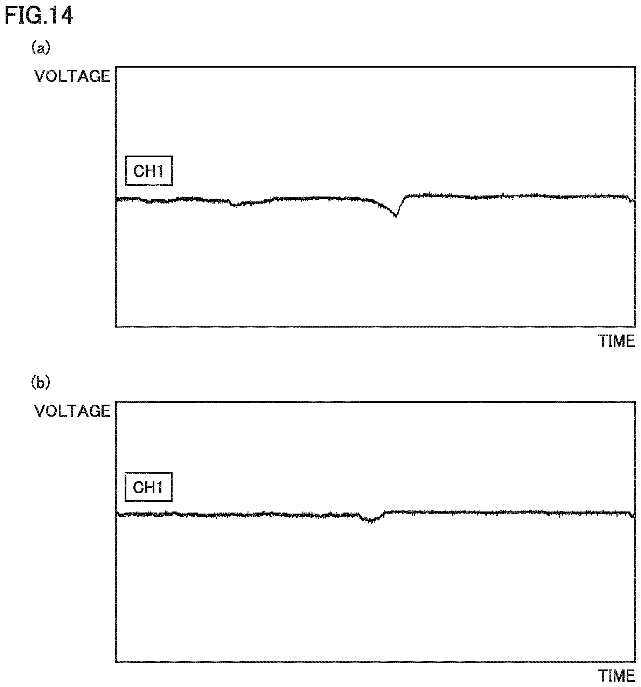

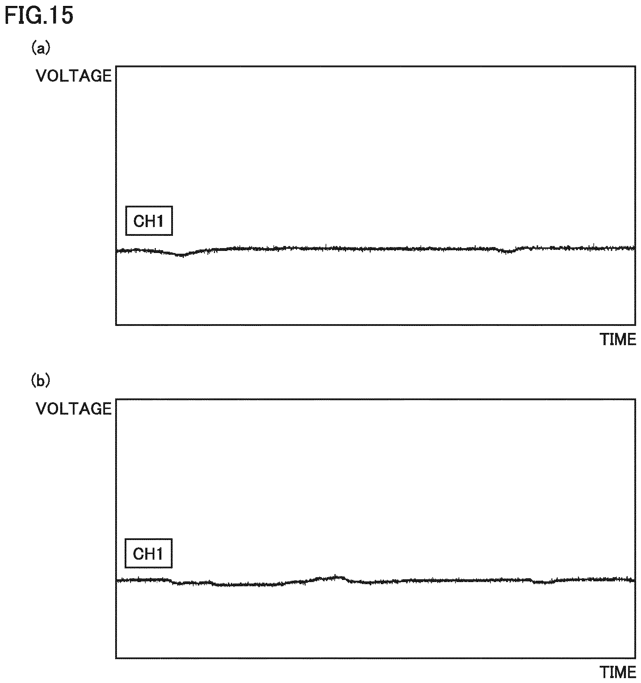

Fig. 13 shows a result of measurement when POF was employed. Fig. 14 shows a result of measurement when GI-SiO₂ was employed. Fig. 15 shows a result of measurement when HSFF was employed. (a) in each figure shows an output waveform from optical fiber sheet 10 when the subject swallowed tea and (b) in each figure shows an output waveform from optical fiber sheet 10 when the subject swallowed almond jelly.

Then, whether the output waveform from optical fiber sheet 10 was varied at the same timing as the output waveform from microphone 20 and magnitude of variation in output waveform from optical fiber sheet 10 were assessed based on the results of measurement.

In assessment, an example in which the output waveform from optical fiber sheet 10 was varied at the same timing as the output waveform from microphone 20 and magnitude of variation was not smaller than a threshold value was determined as "good sensitivity." An example in which the output waveform from optical fiber sheet 10 was not varied at the timing of variation in output waveform from microphone 20 or magnitude of variation in output waveform from optical fiber sheet 10 was smaller than the threshold value was determined as "poor sensitivity." Table 1 shows results of comparison. Table 1 shows sensitivity of each optical fiber with a frequency of assessment as "good sensitivity" of the four results of measurement.

**Table 1**

| Optical Fiber | Frequency of Good Sensitivity |
|---|---|
| POF | 1/4 (25%) |
| GI-SiO₂ | 4/4 (100%) |
| HSFF | 2/4 (50%) |

Referring to Table 1, when POF was employed, though the output waveform from optical fiber sheet 10 was varied, magnitude of variation smaller than the threshold value was observed. Depending on the subject, the output waveform from optical fiber sheet 10 without variation was observed. Consequently, a frequency of good sensitivity was 25%.

When GI-SiO₂ was employed, variation in output waveform from optical fiber sheet 10 was clearly observed in all of the four subjects (frequency of 100%). HSSF exhibited sensitivity intermediate between POF and GI-SiO₂ (frequency of 50%). Consequently, it was confirmed that optical fiber sheet 10 including GI-SiO₂ was highest in sensitivity to swallowing movement.

### [Modification of Embodiment]

A modification of swallowing movement monitoring sensor 1 and swallowing movement monitoring sensor system 200 according to the present embodiment will be described below. Each modification is by way of example and partial substitution or combination of features shown in different modifications can naturally be made.

### [First Modification]

Exemplary placement of an optical fiber on an optical fiber sheet will be described in a first modification.

Figs. 16 to 19 are each a plan view showing exemplary placement of an optical fiber. Optical fiber sheets 10A to 10D shown in Figs. 16 to 19 are all attached around the neck of a subject with the laryngeal portion being defined as the center (see Fig. 2). Optical fiber sheets 10A to 10D are applicable to swallowing movement monitoring sensor system 200 shown in Fig. 7, instead of optical fiber sheet 10.

As described with reference to Fig. 5, optical fiber 14 is placed on sheet-like member 12 as if it were drawn with a single stroke. In exemplary placement shown in Fig. 16, and according to the invention, in optical fiber sheet 10A, a meandering portion formed by deforming one optical fiber portion is placed on sheet-like member 12 as in optical fiber sheet 10 shown in Fig. 5.

Specifically, the meandering portion has a plurality of linear portions 14a which extend in the direction of width (the first direction) of rectangular sheet-like member 12 having a lateral width of 85 mm and a vertical width of 65 mm and a plurality of curved portions 14b connecting end portions of two linear portions 14a to each other.

The plurality of linear portions 14a are arranged as being aligned at an interval in the direction of length (the second direction) of sheet-like member 12. In the example in Fig. 16, and according to the invention, the optical fiber portion is deformed such that a third linear portion 14a located relatively downstream passes between first and second linear portions 14a located relatively upstream of the plurality of linear portions 14a.

According to such a configuration, a length of the optical fiber (an optical path length) in optical fiber sheet 10A is longer than in optical fiber sheet 10. With increase in number of linear portions 14a, an interval between adjacent linear portions 14a is shorter. Consequently, optical fiber sheet 10A is greater in variation in transmission loss in response to movement of the laryngeal portion of the subject than optical fiber sheet 10, and hence it can achieve improved sensitivity as the swallowing movement monitoring sensor.

In exemplary placement shown in Fig. 17, in an optical fiber sheet 10B, a meandering portion formed by deforming one optical fiber portion is placed on sheet-like member 12 as in optical fiber sheet 10. Specifically, the meandering portion has a plurality of linear portions 14a extending in the direction of width of rectangular sheet-like member 12 having a lateral width of 85 mm and a vertical width of 80 mm, a plurality of curved portions 14b connecting end portions of two linear portions 14a to each other, and a plurality of annular portions 14c.

The plurality of annular portions 14c are formed as being aligned in the direction of width of sheet-like member 12 in at least one 14a of the plurality of linear portions 14a. Annular portion 14c does not have to be in a shape of a perfect circle but may be collapsed like an ellipse.

Annular portion 14c is arranged to intersect with an adjacent annular portion 14c and to intersect with at least one linear portion 14a. Intersection means that optical fibers intersect with each other in a plan view of sheet-like member 12 and one optical fiber may apply a lateral pressure to the other optical fiber when force is applied in a direction perpendicular to the surface of sheet-like member 12. The optical fiber produces a transmission loss when a lateral pressure is applied thereto. Therefore, a greater number of intersecting portions is preferable.

In the example in Fig. 17, a contact point where optical fibers are in contact with each other is formed at a portion where annular portions 14c intersect with each other and a portion where annular portion 14c and linear portion 14a intersect with each other. A lateral pressure is produced in optical fiber 14 with the contact point serving as a point of load. Consequently, a higher lateral pressure resulting from movement of the laryngeal portion is produced in optical fiber 14 in optical fiber sheet 10B than in optical fiber sheet 10. Therefore, greater variation in quantity of light is caused in response also to slight movement of the laryngeal portion and hence sensitivity as the swallowing movement monitoring sensor can be improved. A diameter of annular portion 14c, the number of intersecting portions, and a position of the intersecting portion are determined to improve sensitivity of optical fiber sheet 10B in consideration of a material and a geometry of optical fiber 14.

In exemplary placement shown in Figs. 18 and 19, two optical fibers 14U and 14D are placed on sheet-like member 12 as if each of them were drawn with a single stroke. The optical fiber sheet has two input ends 14i1 and 14i2 to which two transmission signal beams are input in parallel and two output ends 14o1 and 14o2 outputting the two transmission signal beams in parallel. A meandering portion formed by deforming one optical fiber portion is placed between an input end and an output end which form a pair.

Specifically, in optical fiber sheet 10C shown in Fig. 18, two meandering portions are placed at an interval in the direction of length (the second direction) of rectangular sheet-like member 12 having a lateral width of 85 mm and a vertical width of 100 mm. One meandering portion is formed by optical fiber 14U and the other meandering portion is formed by optical fiber 14D. Two meandering portions are identical in construction.

In the example in Fig. 18, each meandering portion is constructed similarly to the meandering portion in optical fiber sheet 10A shown in Fig. 16. The meandering portion has a plurality of linear portions 14a extending in the direction of width of sheet-like member 12 and a plurality of curved portions 14b connecting end portions of two linear portions 14a to each other.

In optical fiber sheet 10D shown in Fig. 19, two meandering portions are arranged as being aligned at an interval in the direction of length (the second direction) of rectangular sheet-like member 12 having a lateral width of 85 mm and a vertical width of 75 mm. One meandering portion is formed by optical fiber 14U and the other meandering portion is formed by optical fiber 14D. Two meandering portions are identical in construction.

In the example in Fig. 19, each meandering portion is constructed similarly to the meandering portion in optical fiber sheet 10B shown in Fig. 17. The meandering portion has two linear portions 14a extending in the direction of width of sheet-like member 12, curved portion 14b connecting end portions of two linear portions 14a to each other, and three annular portions 14c formed as being aligned in the direction of width of sheet-like member 12 in linear portion 14a.

In the exemplary placement shown in Figs. 18 and 19, light incident on input ends 14i1 and 14i2 of optical fibers 14U and 14D is transmitted through the optical fibers and thereafter emitted from output ends 14o1 and 14o2. A not-shown optical power meter (light reception portion) is connected to each of output ends 14o1 and 14o2 of optical fibers 14U and 14D.

A lateral pressure in accordance with movement of the laryngeal portion of the subject is applied to the meandering portions of optical fibers 14U and 14D. As the lateral pressure is varied with elevation and descent of the laryngeal portion of the subject, a transmission loss of each of optical fibers 14U and 14D is varied.

In the exemplary placement in Figs. 18 and 19, the meandering portion of optical fiber 14U and the meandering portion of optical fiber 14D are arranged as being aligned in the direction in parallel to movement of the laryngeal portion (the direction shown with the arrow in Fig. 4). Therefore, a transmission loss of optical fiber 14D is varied as being delayed relative to a transmission loss of optical fiber 14U. By detecting this time lag in variation in transmission loss, a speed of elevation of the laryngeal portion (swallowing) of the subject can be measured.

In particular, according to optical fiber sheet 10D shown in Fig. 19, it has been confirmed that a speed of swallowing movement of the laryngeal portion of the subject can accurately be measured even when the subject is an elderly person who is slow in swallowing movement or a female who has a laryngeal portion projecting less than an adult male.

Though Figs. 18 and 19 show constructions in which two optical fibers 14U and 14D are placed on one sheet-like member 12, three or more optical fibers may be placed on sheet-like member 12. Specifically, three or more meandering portions are placed as being aligned at an interval in the direction of length of sheet-like member 12. As a lateral pressure in accordance with movement of the laryngeal portion of the subject is applied to each of the three or more meandering portions, a transmission loss in each of the three or more optical fibers is varied. By detecting a time lag in variation in transmission loss among three or more optical fibers, a speed of swallowing movement can more accurately be measured than in a construction in which a time lag in variation in transmission loss between two optical fibers is detected.

### [Second Modification]

In a second modification, a method of attaching an optical fiber sheet will be described. As shown in Fig. 2, the optical fiber sheet is attached around the neck of a subject with the laryngeal portion being defined as the center. For example, fixing band 300 as shown in Fig. 20 is used for attachment of the optical fiber sheet. A cervical spine fixing instrument for fixing the cervical spine at a constant pressure, a tracheal cannula holder, or a stretchable medical netted bandage is applicable as fixing band 300.

As shown in Fig. 20 (a), optical fiber sheet 10 is mounted on one main surface of fixing band 300. Any of optical fiber sheets 10A to 10D may be mounted instead of optical fiber sheet 10. As shown in Fig. 20 (b), fixing band 300 is bound around the neck of subject 100 such that optical fiber sheet 10 comes in contact with the laryngeal portion. Optical fiber sheet 10 can thus be in intimate contact with the laryngeal portion at a constant pressure.

Swallowing movement of the subject to which optical fiber sheet 10 was attached with fixing band 300 was measured. In measurement, an adult male without dysphagia was adopted as the subject and optical fiber sheet 10 was attached to the laryngeal portion of the subject with the cervical spine fixing instrument.

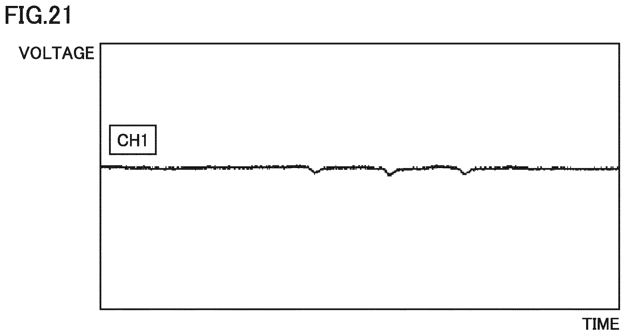

GI-SiO₂ was employed for optical fiber 14 of optical fiber sheet 10. Fig. 21 shows a result of measurement at the time when the subject swallowed saliva consecutively three times. The ordinate in Fig. 21 represents a quantity of received light (voltage) measured with optical power meter 50 as a detection signal from optical fiber sheet 10 and the abscissa in Fig. 21 represents time.

Referring to Fig. 21, CH1 represents an output waveform of a detection signal from optical fiber sheet 10. Three decreases in total in quantity of received light were observed in the output waveform from optical fiber sheet 10. It was confirmed that optical fiber sheet 10 attached with the cervical spine fixing instrument captured movement of the laryngeal portion when the subject swallowed saliva.

Optical fiber sheet 10 can be attached also with an elastic member in a form of a string as shown in Fig. 22, instead of fixing band 300 shown in Fig. 20. Specifically, as shown in Fig. 22 (a), elastic string 310 having a length approximately from 70 to 140 mm is attached to opposing end surfaces in the direction of width of optical fiber sheet 10. Elastic string 310 is in a form of a ring with opposing ends in the direction of length being connected to optical fiber sheet 10. Elastic string 320 is further attached to pass through this ring portion.

As shown in Fig. 22 (b), by winding elastic string 320 around the neck of subject 100, optical fiber sheet 10 is in intimate contact with the laryngeal portion. Any of optical fiber sheets 10A to 10D may be employed instead of optical fiber sheet 10.

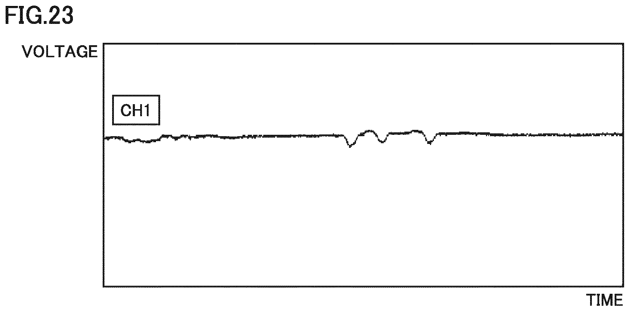

Swallowing movement of the subject to which optical fiber sheet 10 was attached with elastic strings 310 and 320 was measured. The subject is the same as the subject in the results of measurement in Fig. 21. GI-SiO₂ was employed for optical fiber 14 of optical fiber sheet 10. Fig. 23 shows a result of measurement at the time when the subject swallowed saliva consecutively three times. The ordinate in Fig. 23 represents a quantity of received light (voltage) measured with optical power meter 50 as a detection signal from optical fiber sheet 10 and the abscissa in Fig. 23 represents time.

Referring to Fig. 23, CH1 represents an output waveform of a detection signal from optical fiber sheet 10. Three decreases in total in quantity of received light were observed in the output waveform from optical fiber sheet 10. It was confirmed that optical fiber sheet 10 attached with elastic strings 310 and 320 captured movement of the laryngeal portion at the time when the subject swallowed saliva.

When the results of measurement shown in Fig. 21 and the results of measurement shown in Fig. 23 are compared with each other, magnitude of variation exhibited in the output waveform from optical fiber sheet 10 is greater in the results of measurement shown in Fig. 23. Therefore, it was confirmed in the present results of measurement that sensitivity to swallowing movement is higher with optical fiber sheet 10 attached with the elastic member in a form of a string than with optical fiber sheet 10 attached with the fixing band.

### [Third Modification]

Though the configuration in which detection signals from optical fiber sheet 10 and microphone 20 are measured with digital oscilloscope 70 has been described in the embodiment described above, accurate measurement may become difficult due to superimposition of noise generated in digital oscilloscope 70 on the detection signals.

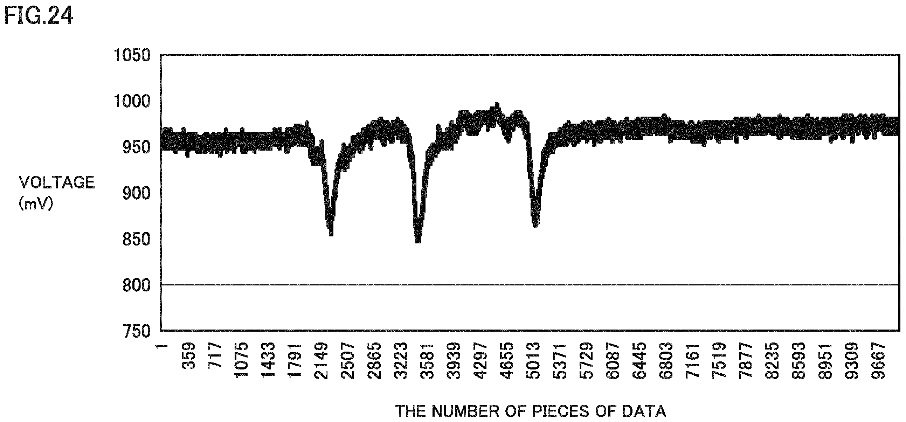

Fig. 24 shows in a graph, a detection signal from optical fiber sheet 10 (voltage waveform data) measured with a digital oscilloscope (a trade name of OWON VDS3104 manufactured by Lilliput) and saved in the CSV format. In Fig. 24, the ordinate represents a quantity of received light (voltage) measured with optical power meter 50 and the abscissa represents the number of pieces of data. Since noise is superimposed on the output waveform, it has become difficult to accurately detect decrease in quantity of received light from the graph.

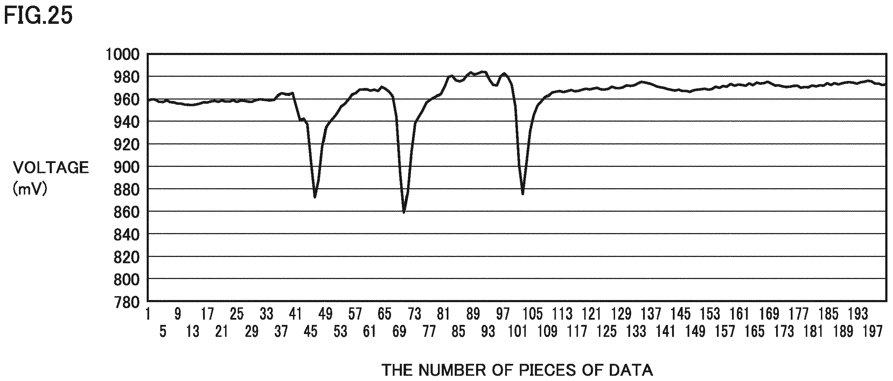

In a third modification, noise superimposed on the output waveform is removed by subjecting the output waveform measured with digital oscilloscope 70 to simple moving average processing. Fig. 25 shows an output waveform from optical fiber sheet 10 subjected to simple moving average processing. The output waveform shown in Fig. 25 is based on a value representing an average of a plurality of (for example, 128) pieces of recent data. It was confirmed that the output waveform shown in Fig. 25 could more clearly express three swallowing movements than the output waveform shown in Fig. 24 as a result of removal of noise. Sensitivity to swallowing movement can thus further be improved.

### [Fourth Modification]

A configuration example of signal processing unit 30 will be described in a fourth modification. Fig. 26 is a schematic configuration diagram of a swallowing movement monitoring sensor system according to the fourth modification.

As shown in Fig. 26, a swallowing movement monitoring sensor system 210 is identical in basic configuration to swallowing movement monitoring sensor system 200 shown in Fig. 7. A difference resides in that an output signal from optical power meter 50 is input to an audio input portion (a first audio input CH1) of PC80 through audio mixer 62 and an output signal from audio mixer 60 is input to an audio input portion (a second audio input CH2) of PC 80 without passing through digital oscilloscope 70.

In the present modification, representation of waveforms of two output signals different in frequency band on PC 80 instead of digital oscilloscope 70 as being aligned on the same time axis was attempted. Namely, PC 80 corresponds to the "display". PC 80 contains an audio input portion configured to be capable of digital sampling of a detection signal from microphone 20. When the display includes no audio input portion, the similar function can be achieved by an external audio input unit.

Specifically, initially, with audio analysis software, an output signal from audio mixer 60 was recorded at a plurality of different sampling frequencies and a lower limit of the sampling frequency at which clicking sound (swallowing sound) was clearly obtained was set. In experiments, clicking sounds recorded at seven sampling frequencies in total of 44.1 kHz, 32 kHz, 22.05 kHz, 16 kHz, 11.025 kHz, 8 kHz, and 4 kHz were compared with one another. It was found from the result of comparison that 16 kHz was the lower limit of the sampling frequency. Then, the sampling frequency was set to 16 kHz.

Then, a dummy signal having a frequency substantially the same as the sampling frequency was superimposed on an output signal from optical power meter 50 having a frequency component around 10 Hz in audio mixer 62.

An output signal from optical power meter 50 (an analog signal output) contains a large amount of direct-current voltage components. Therefore, when an output signal from optical power meter 50 is input to the audio input portion in PC 80 as it is, the direct-current voltage components may be lost. The dummy signal is used as a carrier wave for avoiding such an unfavorable condition.

Specifically, the output signal from optical power meter 50 is converted to an alternating-current signal which can be handled by an audio amplifier as a result of superimposition with a dummy signal. The output signal from optical power meter 50 can thus be input to PC 80 through the audio input function contained in PC 80 or an external audio input unit.

A frequency of the dummy signal is determined based on frequency characteristics of the audio input portion or the audio input unit. Though the frequency of the dummy signal is normally set to a frequency not lower than 16 kHz and not higher than 40 kHz, it should only be within a range permitting audio input so long as the frequency does not affect analysis of swallowing movement, and it may be lower than 16 kHz (for example, 100 Hz).

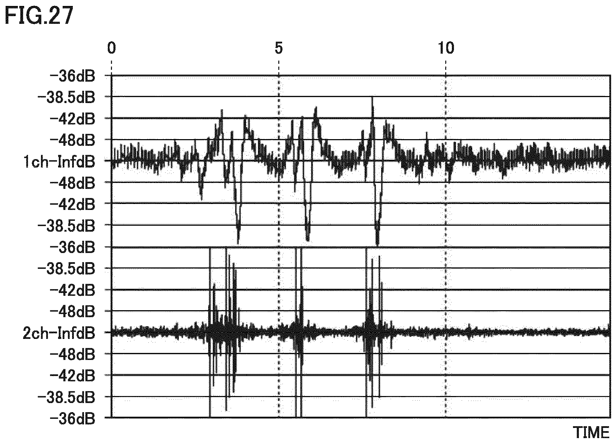

By subjecting the output signal from optical power meter 50 on which the dummy signal had been superimposed and the output signal from audio mixer 60 to digital sampling in the audio input portion of PC 80, they were converted to data in a WAV (resource interface file format (RIFF) waveform audio format) format. The resultant data was shown as being aligned on the same time axis, by using audio analysis software. Fig. 27 shows a result of measurement.

Referring to Fig. 27, CH1 (Ich-InfdB) represents a quantity of received light measured with optical power meter 50 and CH2 (2ch-InfdB) represents an output voltage from a lavaliere condenser microphone. These output waveforms represent results of measurement at the time when one subject swallows saliva consecutively three times.

As shown in Fig. 27, an output signal from optical power meter 50 on which a dummy signal is superimposed is shown with CH1. An output signal from audio mixer 60 is shown with CH2 on the same time axis.

When a frequency of a carrier wave (dummy signal) is the same as the sampling frequency, accurate measurement of the carrier wave must fail in principles. The reason why the carrier wave is shown with CH1 in Fig. 27 may be because there is a slight difference between the sampling frequency and the frequency of the dummy signal or a waveform of the dummy signal is distorted by characteristics of the audio mixer.

Decrease in quantity of received light is observed at timing of observation of the clicking sound also in the present modification and it was confirmed that optical fiber sheet 10 and microphone 20 captured swallowing movement of the subject in good agreement with each other. Thus, a PC with a common audio input function can be used to compare two output waveforms without using an expensive apparatus such as a digital oscilloscope. Therefore, swallowing movement can accurately be detected and analyzed with a simplified apparatus configuration.

### [Fifth Modification]

Other configuration examples of outside shape variation sensor 5 will be described in a fifth modification. As set forth above, outside shape variation sensor 5 is configured to detect variation in outside shape of the laryngeal portion associated with swallowing movement.

A swallowing movement monitoring sensor system 220 shown in Fig. 28 includes a pressure-sensitive sensor sheet 90 as outside shape variation sensor 5.

Pressure-sensitive sensor sheet 90 refers to a pressure-sensitive sensor of a capacitance type formed like a sheet. The pressure-sensitive sensor of the capacitance type converts variation in capacitance caused by deformation of a movable electrode of a capacitor due to an external pressure into an electric signal. In the example in Fig. 28, a pressure applied to the pressure-sensitive sensor is varied with movement of the laryngeal portion of the subject.

Pressure-sensitive sensor sheet 90 converts a measured pressure into an electric signal and outputs the electric signal. An output signal from pressure-sensitive sensor sheet 90 is amplified by an electric amplifier 95 and thereafter a dummy signal is superimposed on the output signal in audio mixer 62. The output signal on which the dummy signal has been superimposed is input to channel CH1 of an audio input of PC 80.

A detection signal indicating swallowing sound detected by microphone 20 is input to channel CH2 of the audio input of PC 80 through audio mixer 60. PC 80 analyzes two pieces of output waveform data input to channels CH1 and CH2 by driving analysis software installed in advance.

A swallowing movement monitoring sensor system 230 shown in Fig. 29 includes a pressure-sensitive rubber sensor sheet 92 as outside shape variation sensor 5.

Pressure-sensitive rubber sensor sheet 92 is pressure-sensitive rubber formed like a sheet. Pressure-sensitive rubber converts variation in electrical resistance value generated as a result of displacement of conductive rubber by an external pressure into an electric signal. In the example in Fig. 29, a pressure applied to pressure-sensitive rubber is varied with movement of the laryngeal portion of the subject.

Pressure-sensitive rubber sensor sheet 92 converts a measured pressure into an electric signal and outputs the electric signal. An output signal from pressure-sensitive rubber sensor sheet 92 is amplified by electric amplifier 95 and thereafter a dummy signal is superimposed on the output signal in audio mixer 62. The output signal on which the dummy signal has been superimposed is input to channel CH1 of the audio input of PC 80.

A detection signal indicating swallowing sound detected by microphone 20 is input to channel CH2 of the audio input of PC 80 through audio mixer 60. PC 80 analyzes two pieces of output waveform data input to channels CH1 and CH2 by driving analysis software installed in advance.

### [Sixth Modification]

Swallowing of food and respiration have been known to be in precise coordination with each other (for example, Harold G. Preiksaitis et al., J Appl Physiol 81: 1707-1714, 1996). According to this publication, normally, a state that respiration temporarily ceases appears at the time of swallowing. The state that respiration temporarily ceases with swallowing is called "swallowing apnea." Swallowing apnea is a function performed for preventing a lump of food from entering a trachea or a bronchus (aspiration).

When the swallowing function is normal, usually, respiration once ceases with transition from exhalation to swallowing and exhalation is again performed after swallowing. A respiration pattern in the order of exhalation → swallowing (apnea) → exhalation is observed. In contrast, when the swallowing function is deteriorated, a respiration pattern before and after swallowing may be disordered. For example, when inhalation is performed instead of exhalation after swallowing, the possibility of aspiration accordingly increases. Therefore, if a respiration pattern in swallowing can accurately and easily be detected with dynamics of swallowing, it may greatly contribute to diagnosis of dysphagia and prevention of aspiration pneumonia.

Currently, inductive plethysmography using Respitrace Calibrator has been adopted for detecting a respiration pattern at the time of swallowing. Since this technique requires a relatively expensive apparatus, it is not necessarily easy to use the technique in general clinical scenes.

The present inventors have succeeded in detecting slight body movement caused by respiration with an optical fiber sheet as an apparatus for diagnosing the sleep apnea syndrome (for example, Seiko Mitachi et al., APSS-SLEEP 2010, Vol. 33, A139, 2010). The diagnosis apparatus can make determination as apnea, hypopnea, or body movement irrelevant to respiration (for example, turning over) based on variation in quantity of light caused by passage through an optical fiber sheet mounted on bedclothing.

Therefore, in a sixth modification, variation in outside shape of the chest associated with swallowing movement is detected with an optical fiber sheet. Specifically, by bringing an optical fiber sheet into press contact with the chest portion of a subject, expansion, contraction, and a still state of the chest, that is, inhalation, exhalation, and a breath cessation period, are observed.

Thus, a respiration pattern in swallowing can be detected in a non-invasive and simplified manner. By combining the detected respiration pattern in swallowing with dynamics of swallowing movement detected by the swallowing movement monitoring sensor according to the embodiment, the swallowing function can objectively be assessed.

Fig. 30 is a diagram showing a configuration of swallowing movement monitoring sensor 1A according to the sixth modification. Referring to Fig. 30, swallowing movement monitoring sensor 1A includes two outside shape variation sensors 5 and 6, microphone 20, and signal processing unit 30.

Swallowing movement monitoring sensor 1A according to the sixth modification is the same as swallowing movement monitoring sensor 1 shown in Fig. 1 to which outside shape variation sensor 6 is added. In the description below, outside shape variation sensor 5 is also referred to as "first outside shape variation sensor 5" and outside shape variation sensor 6 is also referred to as a "second outside shape variation sensor 6."

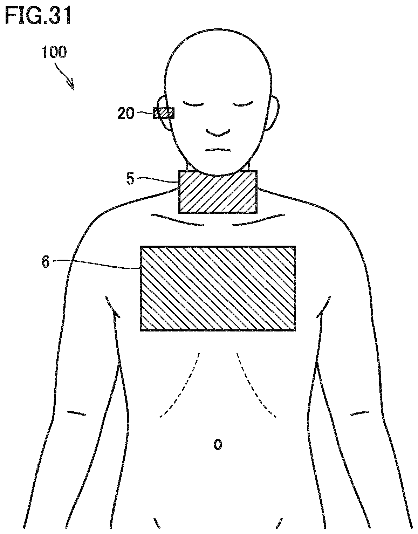

Fig. 31 is a diagram showing attachment of outside shape variation sensors 5 and 6 and microphone 20 shown in Fig. 30 to subject 100. Fig. 31 schematically shows an upper body of subject 100.

First outside shape variation sensor 5 is attached around the neck of subject 100 with the laryngeal portion being defined as the center. The optical fiber sheet (see Fig. 4), the pressure-sensitive sensor sheet (see Fig. 28), or the pressure-sensitive rubber sensor sheet (see Fig. 29) can be employed for first outside shape variation sensor 5. First outside shape variation sensor 5 is configured to detect variation in outside shape of the laryngeal portion associated with swallowing movement.

Microphone 20 is attached to the inside of an ear (in an external auditory meatus) of subject 100. Microphone 20 is configured to detect swallowing sound produced in the ear in association with swallowing movement.

Second outside shape variation sensor 6 is attached to the chest portion of subject 100. An optical fiber sheet is employed for second outside shape variation sensor 6. Second outside shape variation sensor 6 is configured to detect variation in outside shape of the chest associated with swallowing movement. Second outside shape variation sensor 6 may be attached to an abdominal portion instead of or in addition to the chest portion. A pressure-sensitive sensor sheet or a pressure-sensitive rubber sensor sheet may be employed for second outside shape variation sensor 6.

A detection signal indicating variation in outside shape of the laryngeal portion detected by first outside shape variation sensor 5, a detection signal indicating swallowing sound detected by microphone 20, and a detection signal indicating variation in outside shape of the chest detected by second outside shape variation sensor 6 are together input to signal processing unit 30. Signal processing unit 30 processes the detection signal output from each of outside shape variation sensors 5 and 6 and microphone 20.

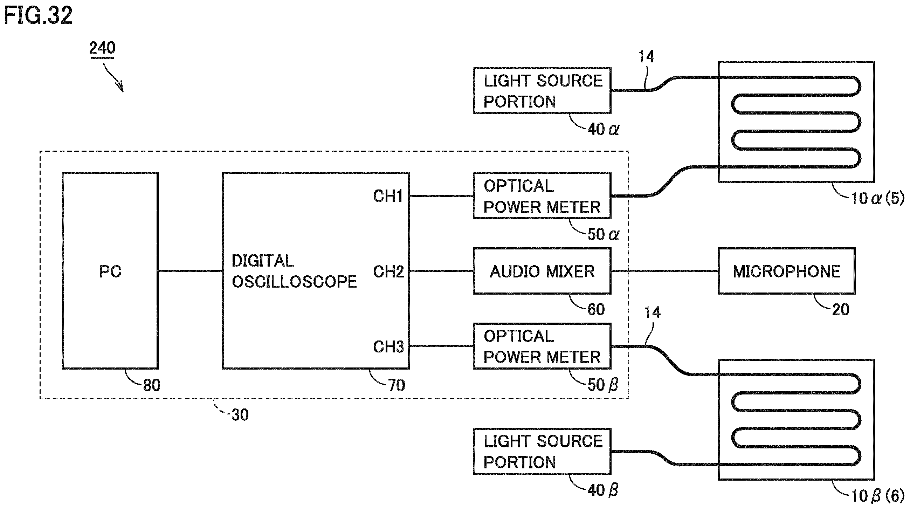

Fig. 32 is a schematic configuration diagram showing one example of a swallowing movement monitoring sensor system configured with swallowing movement monitoring sensor 1A according to the sixth modification.

Referring to Fig. 32, a swallowing movement monitoring sensor system 240 includes an optical fiber sheet 10α (first outside shape variation sensor), an optical fiber sheet 10β (second outside shape variation sensor), microphone 20, light source portions 40α and 40β, optical power meters 50α and 50β, audio mixer 60, digital oscilloscope 70, and PC 80.

As described with reference to Fig. 31, in measuring swallowing movement, optical fiber sheet 10α representing first outside shape variation sensor 5 is attached to the laryngeal portion of the subject and microphone 20 is attached to the inside of the ear of the subject. In measuring a respiration pattern in swallowing, optical fiber sheet 10β representing second outside shape variation sensor 6 is attached to the chest portion of the subject. A method of attaching optical fiber sheet 10β is the same as the method of attaching optical fiber sheet 10α.

Optical fiber sheets 10α and 10β are identical in basic construction to optical fiber sheets 10 and 10A to 10D in the embodiments above. As shown in Figs. 5 and 16 to 19, one optical fiber 14 or a plurality of optical fibers 14 is/are placed on a rectangular sheet-like member as if it/they were drawn with a single stroke.

A constant quantity of light continuously supplied from light source portion 40α is incident on the input end of optical fiber 14 of optical fiber sheet 10α. Optical power meter 50α receives emission light transmitted through optical fiber 14 and measures a quantity of received light. The measured quantity of received light (an amount of attenuation) is varied in accordance with a lateral pressure applied to optical fiber sheet 10α with movement of the laryngeal portion of the subject. Optical power meter 50α converts the measured quantity of received light into an electric signal and inputs the resultant electric signal to channel CH1 of digital oscilloscope 70.

A constant quantity of light continuously supplied from light source portion 40β is incident on the input end of optical fiber 14 of optical fiber sheet 10β. Optical power meter 50β receives emission light transmitted through optical fiber 14 and measures a quantity of received light. The measured quantity of received light (an amount of attenuation) is varied in accordance with a lateral pressure applied to optical fiber sheet 10β with movement of the chest of the subject. Optical power meter 50β converts the measured quantity of received light into an electric signal and inputs the resultant electric signal to a channel CH3 of digital oscilloscope 70.

Variation in quantity of received light (an amount of attenuation) measured with optical fiber sheet 10β is substantially in proportion to a lateral pressure applied to optical fiber 14 with movement of the chest. Therefore, it can be determined that movement of the chest is small when variation in quantity of received light is small and movement of the chest is great when variation in quantity of received light is great. In the present modification, expansion, contraction, and a still state of the chest, that is, inhalation, exhalation, and a breath cessation period, are determined based on variation in quantity of received light.

A detection signal indicating swallowing sound detected by microphone 20 is input to audio mixer 60. Audio mixer 60 adjusts a volume of the detected swallowing sound and inputs the adjusted detection signal to channel CH2 of digital oscilloscope 70.

Digital oscilloscope 70 receives an output signal from optical power meter 50α at channel CHI, receives an output signal from audio mixer 60 at channel CH2, and receives an output signal from optical power meter 50β at channel CH3. Digital oscilloscope 70 holds variation over time (output waveform) of the output signals from optical power meters 50α and 50β and the output signal from audio mixer 60, for example, as voltage waveform data. Digital oscilloscope 70 transmits three output waveforms to PC 80.

Digital oscilloscope 70 can also show the three output waveforms as being aligned on the same time axis. Digital oscilloscope 70 is configured to be able to show the output waveform of the detection signal from microphone 20 and the output waveforms of the detection signals from optical fiber sheets 10α and 10β as being aligned on the same time axis.

PC 80 analyzes three pieces of output waveform data transmitted from digital oscilloscope 70 by driving analysis software installed in advance. A result of analysis by PC 80 is shown on a display apparatus (not shown) such as a CRT or a printer.

According to the configuration as above, swallowing movement monitoring sensor system 240 can simultaneously and non-invasively capture variation in outside shape of the laryngeal portion and swallowing sound involved with swallowing movement of the subject as well as variation in outside shape of the chest involved with swallowing movement with a simplified configuration. Thus, swallowing movement and a respiration pattern in swallowing can non-invasively and objectively be measured. Consequently, the swallowing function can objectively be assessed even in facilities lacking sufficient medical equipment or staff and hence great contribution to development of medical care can be achieved.

It should be understood that the embodiments above are illustrative and non-restrictive in every respect. The scope of the present invention is defined by the claims rather than the description above and is intended to include any modifications within the scope of the claims.

### REFERENCE SIGNS LIST

1, 1A swallowing movement monitoring sensor; 10, 10A to 10D, 10α, 10β optical fiber sheet; 12 sheet-like member; 14 optical fiber; 14a linear portion; 14b curved portion; 14c annular portion; 14i, 14i1, 14i2 input end; 14o, 14o1, 14o2 output end; 20 microphone; 30 signal processing unit; 40, 40α, 40β light source portion; 50, 50α, 50β optical power meter; 60, 62 audio mixer; 64 dummy signal generator; 70 digital oscilloscope; 80 PC; 90 pressure-sensitive sensor sheet; 92 pressure-sensitive rubber sensor sheet; 95 electric amplifier; 100 subject; 102 nasal cavity; 104 tongue; 106 pharyngeal portion; 108 laryngeal portion; 110 trachea; 112 esophagus; 114 ear; 116 external auditory meatus; 200, 210, 220, 230, 240 swallowing movement monitoring sensor system

## Claims

1. A swallowing movement monitoring sensor (1) which is configured to measure swallowing movement of a subject (100) comprising a first outside shape variation sensor (5) configured to be attached to a laryngeal portion (108) of the subject (100),
the first outside shape variation sensor (5) being configured to detect variation in outside shape of the laryngeal portion (108) associated with swallowing movement,
wherein the first outside shape variation sensor (5) includes an optical fiber sheet (10) attached to the laryngeal portion (108), the optical fiber sheet (10) having an optical fiber (14) and a sheet-like member (12) supporting the optical fiber (14),
wherein the optical fiber sheet (10) is configured to detect variation in quantity of light of a transmission signal beam based on a loss produced by a lateral pressure applied to the optical fiber (14) from the laryngeal portion (108) in association with variation in outside shape of the laryngeal portion (108) involved with swallowing movement,
**characterized in that** the optical fiber (14) has a meandering portion formed from one optical fiber portion, the meandering portion comprising a plurality of linear portions (14a) extending in a first direction and a plurality of curved portions (14b) connecting end portions of two linear portions (14a) to each other, and
the optical fiber portion comprising a third linear portion (14a) located relatively downstream which passes between first and second linear portions (14a) located relatively upstream in the meandering portion.

2. The swallowing movement monitor sensor (1) according to claim 1, wherein an interval between adjacent linear portions (14a) is such that a lateral pressure is applied to at least two linear portions (14a) in response to movement of the laryngeal portion (108).

3. The swallowing movement monitoring sensor (1) according to claim 1, wherein
the optical fiber portion comprises a plurality of annular portions (14c) aligned in the first direction which are further formed in at least one of the plurality of linear portions (14a) in the meandering portion.

4. The swallowing movement monitoring sensor (1) according to claim 1 or claim 3, wherein
the optical fiber sheet (10) further includes a plurality of input ends (14i1, 14i2) adapted to receive a plurality of transmission signal beams as input in parallel and a plurality of output ends (14o1, 14o2) adapted to output the plurality of transmission signal beams in parallel, and
the optical fiber portion is placed between the input end (14il, 14i2) and the output end (14o1, 14o2) which form a pair.

5. The swallowing movement monitoring sensor (1) according to any one of claims 1, 3 or 4, wherein
the optical fiber (14) is a plastic optical fiber, a quartz-based optical fiber, or a plastic clad quartz glass core optical fiber.

6. The swallowing movement monitoring sensor (1) according to claim 5, wherein
the optical fiber (14) is of a step index type, a graded index type, or a single mode type.

7. The swallowing movement monitoring sensor (1) according to claim 1 or claims 3 to 6, wherein
the optical fiber sheet (10) has a width in the first direction not smaller than 30 mm and not greater than 500 mm, and a width in a second direction perpendicular to the first direction not smaller than 30 mm and not greater than 150 mm.

8. The swallowing movement monitoring sensor (1) according to claim 7, wherein
the optical fiber sheet (10) is attachable around a neck, with the laryngeal portion (108) being defined as a center, with a fixing band (300).

9. The swallowing movement monitoring sensor (1) according to claim 7, wherein
the optical fiber sheet (10) is attachable around a neck, with the laryngeal portion (108) being defined as a center, with an elastic member in a form of a string attached to opposing lateral side surfaces of the optical fiber sheet (10).

10. The swallowing movement monitoring sensor (1) according to claim 7, wherein
the optical fiber sheet (10) is attachable around a neck, with the laryngeal portion (108) being defined as a center, with an annular net made of a stretchable material.

11. The swallowing movement monitoring sensor (1) according to any one of claims 1 or 3 to 10, further comprising a microphone (20) attachable in an ear (114) of the subject (100), wherein
the microphone (20) is configured to detect swallowing sound produced in the ear (114) in association with swallowing movement.

12. The swallowing movement monitoring sensor (1) according to claim 11, wherein
the microphone (20) is implemented by a lavaliere condenser microphone having a diameter not smaller than 1.5 mm and not greater than 5.0 mm.

13. The swallowing movement monitoring sensor (1) according to claim 11 or 12, further comprising a signal processing unit (30) configured to process a detection signal output from each of the first outside shape variation sensor (5) and the microphone (20).

14. The swallowing movement monitoring sensor (1) according to claim 13, wherein
the signal processing unit (30) includes a display configured to show an output waveform of the detection signal from the microphone (20) and an output waveform of the detection signal from the first outside shape variation sensor (5) as being aligned on an identical time axis.

15. The swallowing movement monitoring sensor (1) according to claim 14, wherein
the display is configured to show the output waveform obtained by subjecting the detection signal from the first outside shape variation sensor (5) to simple moving average processing.

16. The swallowing movement monitoring sensor (1) according to claim 14 or 15, wherein
the signal processing unit (30) further includes an audio input portion configured to perform digital sampling of the detection signal from the microphone (20),
the audio input portion receives input of the detection signal from the first outside shape variation sensor (5) on which a dummy signal having a frequency handleable by the audio input portion is superimposed, together with the detection signal from the microphone (20), and
output waveforms of the detection signal from the microphone (20) and the detection signal from the first outside shape variation sensor (5) are subjected to digital sampling in the audio input portion and thereafter shown on the display as being aligned on the identical time axis.

17. The swallowing movement monitoring sensor (1) according to claim 1, the swallowing movement monitoring sensor (1) further comprising a second outside shape variation sensor (6) attachable to a chest portion or an abdominal portion of the subject (100), wherein
the second outside shape variation sensor (6) is configured to detect variation in outside shape of a chest associated with swallowing movement.

18. The swallowing movement monitoring sensor (1) according to claim 17, wherein
the second outside shape variation sensor (6) includes an optical fiber sheet (10) having an optical fiber (14) and a sheet-like member (12) supporting the optical fiber (14) and attached to the chest portion or the abdominal portion, and
the optical fiber sheet (10) is configured to detect variation in quantity of light of a transmission signal beam based on a loss produced by a lateral pressure applied to the optical fiber (14) from the chest portion or the abdominal portion in association with variation in outside shape of the chest involved with swallowing movement.

## Patentansprüche

1. Schluckbewegungsüberwachungssensor (1), der konfiguriert ist, um die Schluckbewegung einer Person (100) zu messen, der einen ersten Außenformveränderungssensor (5) umfasst, der konfiguriert ist, um an einem Kehlkopfteil (108) der Person (100) angebracht zu werden,
wobei der erste Außenformveränderungssensor (5) konfiguriert ist, um eine Veränderung der Außenform des Kehlkopfteils (108) in Verbindung mit der Schluckbewegung zu erfassen,
wobei der erste Außenformveränderungssensor (5) einen am Kehlkopfteil (108) angebrachten Lichtleiterbogen (10) aufweist, wobei die Lichtleiterbogen (10) einen Lichtleiter (14) und ein den Lichtleiter (14) tragendes bogenartiges Element (12) aufweist, wobei
der Lichtleiterbogen (10) konfiguriert ist, um eine Änderung der Lichtmenge eines Übertragungssignalstrahls auf der Basis eines Verlustes zu erfassen, der durch einen auf den Lichtleiter (14) vom Kehlkopfteil (108) ausgeübten seitlichen Druck in Verbindung mit einer Änderung der Außenform des Kehlkopfteils (108) erzeugt wird, die mit einer Schluckbewegung verbunden ist,
**dadurch gekennzeichnet, dass**
der Lichtleiter (14) einen mäandernden Teil hat, der aus einem Lichtleiterteil gebildet ist, wobei der mäandernde Teil eine Vielzahl von linearen Teilen (14a), die sich in einer ersten Richtung erstrecken, und eine Vielzahl von gekrümmten Teilen (14b) umfasst, die Endteile von zwei linearen Teilen (14a) miteinander verbinden, und
wobei der Lichtleiterteil einen dritten linearen relativ stromabwärts befindlichen Teil (14a) umfasst, der zwischen einem ersten und einem zweiten linearen Teil (14a) verläuft, die sich relativ stromaufwärts im mäandernden Teil befinden.

2. Schluckbewegungsüberwachungssensor (1) nach Anspruch 1, wobei ein Intervall zwischen benachbarten linearen Teilen (14a) derart ist, dass ein seitlicher Druck auf wenigstens zwei lineare Teile (14a) in Reaktion auf eine Bewegung des Kehlkopfteils (108) ausgeübt wird.

3. Schluckbewegungsüberwachungssensor (1) nach Anspruch 1, wobei der Lichtleiterteil eine Vielzahl von ringförmigen in der ersten Richtung ausgerichteten Teilen (14c) umfasst, die des Weiteren in wenigstens einem aus der Vielzahl von linearen Teilen (14a) in dem mäanderförmigen Teil ausgebildet sind.

4. Schluckbewegungsüberwachungssensor (1) nach Anspruch 1 oder Anspruch 3, wobei
der Lichtleiterbogen (10) des Weiteren eine Vielzahl von Eingangsenden (14i1, 14i2), die angepasst sind, um eine Vielzahl von Übertragungssignalstrahlen als parallele Eingabe zu empfangen, und eine Vielzahl von Ausgangsenden (14o1, 14o2) aufweist, die angepasst sind, um die Vielzahl von Übertragungssignalstrahlen parallel auszugeben, wobei der Lichtleiterteil zwischen dem Eingangsende (14i1, 14i2) und dem Ausgangsende (14o1, 14o2) platziert ist, die ein Paar bilden.

5. Schluckbewegungsüberwachungssensor (1) nach einem der Ansprüche 1, 3 oder 4, wobei
der Lichtleiter (14) ein Lichtleiter aus Kunststoff, ein Lichtleiter auf Quarzbasis oder ein Lichtleiter mit einem kunststoffummantelten Quarzglaskern ist.

6. Schluckbewegungsüberwachungssensor (1) nach Anspruch 5, wobei der Lichtleiter (14) von einem Stufenindextyp, einem Gradientenindextyp oder einem Einzelmodustyp ist.

7. Schluckbewegungsüberwachungssensor (1) nach Anspruch 1 oder einem der Ansprüche 3 bis 6, wobei der Lichtleiterbogen (10) eine Breite in der ersten Richtung von nicht weniger als 30 mm und nicht mehr als 500 mm und eine Breite in einer zweiten Richtung rechtwinklig zu der ersten Richtung von nicht weniger als 30 mm und nicht mehr als 150 mm hat.

8. Schluckbewegungsüberwachungssensor (1) nach Anspruch 7, wobei der Lichtleiterbogen (10) mit einem Fixierband (300) um den Hals anbringbar ist, wobei der Kehlkopfteil (108) als eine Mitte definiert ist.

9. Schluckbewegungsüberwachungssensor (1) nach Anspruch 7, wobei der Lichtleiterbogen (10) um den Hals anbringbar ist, wobei der Kehlkopfteil (108) als eine Mitte definiert ist, mit einem elastischen Element in der Form einer Schnur, die an gegenüberliegenden Seitenflächen des Lichtleiterbogens (10) angebracht wird.

10. Schluckbewegungsüberwachungssensor (1) nach Anspruch 7, wobei der Lichtleiterbogen (10) um den Hals anbringbar ist, wobei der Kehlkopfteil (108) als eine Mitte definiert ist, mit einem ringförmigen Netz, das aus einem dehnbaren Material besteht.

11. Schluckbewegungsüberwachungssensor (1) nach einem der Ansprüche 1 oder 3 bis 10, der des Weiteren ein Mikrofon (20) umfasst, das in einem Ohr (114) der Person (100) anbringbar ist, wobei das Mikrofon (20) konfiguriert ist, um Schluckgeräusche zu erfassen, die im Ohr (114) in Verbindung mit der Schluckbewegung erzeugt werden.

12. Schluckbewegungsüberwachungssensor (1) nach Anspruch 11, wobei das Mikrofon (20) durch ein Lavalier-Kondensatormikrofon mit einem Durchmesser von nicht weniger als 1,5 mm und nicht mehr als 5,0 mm implementiert wird.

13. Schluckbewegungsüberwachungssensor (1) nach Anspruch 11 oder 12, der des Weiteren eine Signalverarbeitungseinheit (30) umfasst, die konfiguriert ist, um ein Detektionssignal zu verarbeiten, das jeweils von dem ersten Außenformveränderungssensor (5) und dem Mikrofon (20) ausgegeben wird.

14. Schluckbewegungsüberwachungssensor (1) nach Anspruch 13, wobei die Signalverarbeitungseinheit (30) ein Display aufweist, das konfiguriert ist, um eine Ausgangswellenform des Detektionssignals vom Mikrofon (20) und eine Ausgangswellenform des Detektionssignals vom ersten Außenformveränderungssensor (5) so anzuzeigen, dass sie auf einer identischen Zeitachse ausgerichtet sind.

15. Schluckbewegungsüberwachungssensor (1) nach Anspruch 14, wobei das Display konfiguriert ist, um die Ausgangswellenform zu zeigen, die gewonnen wird, indem das Detektionssignal vom ersten Außenformveränderungssensor (5) einer einfachen Bewegungsdurchschnittsverarbeitung unterzogen wird.

16. Schluckbewegungsüberwachungssensor (1) nach Anspruch 14 oder 15, wobei
die Signalverarbeitungseinheit (30) des Weiteren einen Audio-Eingabeteil aufweist, der konfiguriert ist, um eine digitale Abtastung des Detektionssignals von dem Mikrofon (20) durchzuführen,
der Audio-Eingabeteil eine Eingabe des Detektionssignals von dem ersten Außenformveränderungssensor (5) empfängt, auf dem ein Leersignal mit einer Frequenz, die von dem Audio-Eingabeteil verarbeitet werden kann, zusammen mit dem Detektionssignal vom Mikrofon (20) überlagert wird, und
Ausgangswellenformen des Detektionssignals vom Mikrofon (20) und des Detektionssignals vom ersten Außenformveränderungssensor (5) einer digitalen Abtastung in dem Audio-Eingabeteil unterzogen und danach auf dem Display so gezeigt werden, dass sie auf der identischen Zeitachse ausgerichtet sind.

17. Schluckbewegungsüberwachungssensor (1) nach Anspruch 1, wobei der Schluckbewegungsüberwachungssensor (1) des Weiteren einen zweiten Außenformveränderungssensor (6) umfasst, der an einem Brustteil oder einem Bauchteil des Person (100) anbringbar ist, wobei
der zweite Außenformveränderungssensor (6) konfiguriert ist, um die Veränderung der Außenform eines Brustkorbs im Zusammenhang mit einer Schluckbewegung zu erfassen.

18. Schluckbewegungsüberwachungssensor (1) nach Anspruch 17, wobei der zweite Außenformveränderungssensor (6) einen Lichtleiterbogen (10) mit einem Lichtleiter (14) und einem bogenartigen Element (12) aufweist, das den Lichtleiter (14) trägt, und an dem Brustteil oder dem Bauchteil angebracht ist, und
der Lichtleiterbogen (10) konfiguriert ist, um eine Änderung der Lichtmenge eines Übertragungssignalstrahls auf der Basis eines Verlustes zu erfassen, der durch einen auf den Lichtleiter (14) vom Brustteil oder Bauchteil ausgeübten seitlichen Druck in Verbindung mit einer Änderung der Außenform der Brust erzeugt wird, die mit einer Schluckbewegung verbunden ist.

## Revendications

1. Capteur de surveillance de mouvement de déglutition (1) qui est configuré de manière à mesurer le mouvement de déglutition d'un sujet (100) comprenant un premier capteur de variation de forme extérieure (5) configuré de manière à être fixé à une partie laryngée (108) du sujet (100),
le premier capteur de variation de forme extérieure (5) étant configuré de manière à détecter une variation de forme extérieure de la partie laryngée (108) associée au mouvement de déglutition ;
dans lequel le premier capteur de variation de forme extérieure (5) inclut une feuille de fibre optique (10) fixée à la partie laryngée (108), la feuille de fibre optique (10) présentant une fibre optique (14) et un élément en forme de feuille (12) prenant en charge la fibre optique (14) ;
dans lequel la feuille de fibre optique (10) est configurée de manière à détecter une variation de quantité de lumière d'un faisceau de signal de transmission sur la base d'une perte produite par une pression latérale appliquée à la fibre optique (14) à partir de la partie laryngée (108) conjointement avec une variation de forme extérieure de la partie laryngée (108) impliquée dans le mouvement de déglutition ;
**caractérisé en ce que** :
la fibre optique (14) présente une partie en méandres formée à partir d'une partie de fibre optique, la partie en méandres comprenant une pluralité de parties linéaires (14a) s'étendant dans une première direction et une pluralité de parties incurvées (14b) reliant des parties d'extrémité de deux parties linéaires (14a) l'une à l'autre ; et
la partie de fibre optique comprenant une troisième partie linéaire (14a) située relativement en aval qui passe entre la première partie linéaire et la deuxième partie linéaire (14a) situées relativement en amont dans la partie en méandres.

2. Capteur de surveillance de mouvement de déglutition (1) selon la revendication 1, dans lequel un intervalle entre des parties linéaires adjacentes (14a) est tel qu'une pression latérale est appliquée à au moins deux parties linéaires (14a) en réponse au mouvement de la partie laryngée (108).

3. Capteur de surveillance de mouvement de déglutition (1) selon la revendication 1, dans lequel la partie de fibre optique comprend une pluralité de parties annulaires (14c) alignées dans la première direction, lesquelles sont en outre formées dans au moins une partie parmi la pluralité de parties linéaires (14a) dans la partie en méandres.

4. Capteur de surveillance de mouvement de déglutition (1) selon la revendication 1 ou 3, dans lequel :
la feuille de fibre optique (10) inclut en outre une pluralité d'extrémités d'entrée (14i1, 14i2) apte à recevoir en entrée une pluralité de faisceaux de signaux de transmission en parallèle, et une pluralité d'extrémités de sortie (14o1, 14o2) apte à fournir en sortie la pluralité de faisceaux de signaux de transmission en parallèle ; et
la partie de fibre optique est placée entre l'extrémité d'entrée (14i1, 14i2) et l'extrémité de sortie (14o1, 14o2) lesquelles forment une paire.

5. Capteur de surveillance de mouvement de déglutition (1) selon l'une quelconque des revendications 1, 3 et 4, dans lequel :
la fibre optique (14) est une fibre optique en plastique, une fibre optique à base de quartz ou une fibre optique à cœur en verre de quartz et à gaine de plastique.

6. Capteur de surveillance de mouvement de déglutition (1) selon la revendication 5, dans lequel :
la fibre optique (14) est de type à saut d'indice, de type à indice gradué ou de type monomode.

7. Capteur de surveillance de mouvement de déglutition (1) selon la revendication 1 ou selon l'une quelconque des revendications 3 à 6, dans lequel :
la feuille de fibre optique (10) présente une largeur, dans la première direction, qui n'est pas inférieure à 30 mm et qui n'est pas supérieure à 500 mm, et une largeur, dans une seconde direction, perpendiculaire à la première direction, qui n'est pas inférieure à 30 mm et qui n'est pas supérieure à 150 mm.

8. Capteur de surveillance de mouvement de déglutition (1) selon la revendication 7, dans lequel :
la feuille de fibre optique (10) peut être fixée autour d'un cou, où la partie laryngée (108) est définie en tant que centre, avec une bande de fixation (300).

9. Capteur de surveillance de mouvement de déglutition (1) selon la revendication 7, dans lequel :
la feuille de fibre optique (10) peut être fixée autour d'un cou, où la partie laryngée (108) est définie en tant que centre, avec un élément élastique en forme de chaîne fixé à des surfaces latérales opposées de la feuille de fibre optique (10).

10. Capteur de surveillance de mouvement de déglutition (1) selon la revendication 7, dans lequel :
la feuille de fibre optique (10) peut être fixée autour d'un cou, où la partie laryngée (108) est définie en tant que centre, avec un filet annulaire composé d'un matériau étirable.

11. Capteur de surveillance de mouvement de déglutition (1) selon l'une quelconque des revendications 1 ou 3 à 10, comprenant en outre un microphone (20) pouvant être fixé dans une oreille (114) du sujet (100), dans lequel :
le microphone (20) est configuré de manière à détecter un son de déglutition produit dans l'oreille (114) conjointement avec un mouvement de déglutition.

12. Capteur de surveillance de mouvement de déglutition (1) selon la revendication 11, dans lequel :
le microphone (20) est mis en œuvre par un microphone à condensateur lavallière dont le diamètre n'est pas inférieur à 1,5 mm et n'est pas supérieur à 5,0 mm.

13. Capteur de surveillance de mouvement de déglutition (1) selon la revendication 11 ou 12, comprenant en outre une unité de traitement de signal (30) configurée de manière à traiter un signal de détection fourni en sortie à partir de chacun parmi le premier capteur de variation de forme extérieure (5) et le microphone (20).

14. Capteur de surveillance de mouvement de déglutition (1) selon la revendication 13, dans lequel :
l'unité de traitement de signal (30) inclut un affichage configuré de manière à montrer une forme d'onde de sortie du signal de détection provenant du microphone (20) et une forme d'onde de sortie du signal de détection provenant du premier capteur de variation de forme extérieure (5) comme étant alignées sur un axe temporel identique.

15. Capteur de surveillance de mouvement de déglutition (1) selon la revendication 14, dans lequel :
l'affichage est configuré de manière à montrer la forme d'onde de sortie obtenue en soumettant le signal de détection, provenant du premier capteur de variation de forme extérieure (5), à un traitement de moyenne mobile simple.

16. Capteur de surveillance de mouvement de déglutition (1) selon la revendication 14 ou 15, dans lequel :
l'unité de traitement de signal (30) inclut en outre une partie d'entrée audio configurée de manière à mettre en œuvre un échantillonnage numérique du signal de détection provenant du microphone (20) ;
la partie d'entrée audio reçoit une entrée du signal de détection en provenance du premier capteur de variation de forme extérieure (5), sur lequel est superposé un signal fictif dont la fréquence peut être gérée par la partie d'entrée audio, conjointement avec le signal de détection provenant du microphone (20) ; et
des formes d'onde de sortie du signal de détection provenant du microphone (20) et du signal de détection provenant du premier capteur de variation de forme extérieure (5) sont soumises à un échantillonnage numérique dans la partie d'entrée audio et sont ensuite montrées sur l'affichage comme étant alignées sur un axe temporel identique.

17. Capteur de surveillance de mouvement de déglutition (1) selon la revendication 1, dans lequel le capteur de surveillance de mouvement de déglutition (1) comprend en outre un second capteur de variation de forme extérieure (6) pouvant être fixé à une partie thoracique ou à une partie abdominale du sujet (100), dans lequel :
le second capteur de variation de forme extérieure (6) est configuré de manière à détecter une variation de forme extérieure d'une poitrine associée au mouvement de déglutition.

18. Capteur de surveillance de mouvement de déglutition (1) selon la revendication 17, dans lequel :
le second capteur de variation de forme extérieure (6) inclut une feuille de fibre optique (10) présentant une fibre optique (14) et un élément en forme de feuille (12) prenant en charge la fibre optique (14) et fixé à la partie thoracique ou à la partie abdominale ; et
la feuille de fibre optique (10) est configurée de manière à détecter une variation de quantité de lumière d'un faisceau de signal de transmission sur la base d'une perte produite par une pression latérale appliquée à la fibre optique (14), à partir de la partie thoracique ou de la partie abdominale, conjointement avec une variation de forme extérieure du thorax impliqué dans le mouvement de déglutition.
